# FASCICULE DE BREVET EUROPEEN

(11) **EP 3 523 624 B1**
(45) Date de publication et mention de la délivrance du brevet: **27.03.2024**
(21) Numéro de dépôt: 17794362.8
(22) Date de dépôt: 09.10.2017
(51) Int. Cl.: G01N 1/40, C12Q 1/24, C12M 1/26, B01L 3/00, C12M 1/00

(54) **PROCÉDÉ ET DISPOSITIF DE PRÉPARATION D'ÉCHANTILLONS**
VERFAHREN UND VORRICHTUNG ZUR VORBEREITUNG VON PROBEN
METHOD AND DEVICE FOR PREPARING SAMPLES

(30) Priorité: 10.10.2016 FR 1659745; 10.10.2016 FR 1659746
(43) Date de publication de la demande: 14.08.2019
(73) Titulaire: Biomérieux, 69280 Marcy l'Étoile (FR)
(72) Inventeur: DACHAUD, Fabien, 25160 Oye-et-Pallet (FR); DACHAUD, Jacques, 25000 Besançon (FR); FOUCAULT, Frédéric, 69280 Marcy L'Etoile (FR); MINASSIAN, Edgar, 69210 Lentilly (FR); MOSTICONE, David, 69280 Sainte Concorce (FR); RAYMOND, Jean-Claude, 69690 Bessenay (FR)
(74) Mandataire: bioMérieux PI Groupement mandataires
(86) Numéro de dépôt international: PCT/FR2017/052755
(87) Numéro de publication internationale: WO 2018/069612

(56) Documents cités:
- CA-A1- 2 264 206
- US-A- 5 976 824
- US-A1- 2011 294 205
- US-A1- 2013 071 872

## Description

### Domaine technique

La présente invention concerne, de manière générale, la préparation d'échantillon aux fins de capture et concentration de microorganisme ou de protéine issue dudit microorganisme. Le domaine d'application de l'invention est celui de la microbiologie et plus particulièrement celui de la microbiologie industrielle.

### Etat de la technique

Les produits bruts utilisés et les produits transformés commercialisés par l'industrie agroalimentaire (produits camés, produits laitiers, produits de la mer, végétaux etc.), ainsi que les produits pharmaceutiques et cosmétiques et l'eau destinée à la boisson, sont soumis à de nombreux tests microbiologiques afin de s'assurer de leur innocuité (absence de bactéries pathogènes ou de dégradation, absence de bactéries marqueurs de contamination dangereuse pour la santé, absence de toxines).

L'analyse microbiologique de ces produits nécessite des techniques précises dont le temps d'obtention du résultat doit être le plus court possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale dans le domaine vétérinaire.

Dans le domaine alimentaire, la problématique est identique. Elle distingue cependant :
- les micro-organismes pathogènes (tels que les Salmonella ou la souche E. Coli 0157 :H7) et leurs toxines dont la recherche s'applique aux matières premières, produits intermédiaires, produits finis commercialisés,
- les micro-organismes non pathogènes, utilisés comme indicateurs-qualité du processus de production, des matières premières aux produits finis, tout au long de la chaîne,
- les bactéries d'intérêt technologique telles que les ferments,
- les micro-organismes marqueurs de contamination.

La détection rapide et précise des contaminations présumées (au sein des lots alimentaires) permet de les contrôler et d'engager ainsi à bref délai des actions correctives.

Techniquement, l'analyse microbiologique met généralement en oeuvre une ou plusieurs phases de pré-enrichissement et/ou d'enrichissement, une ou plusieurs phases de détection, une ou plusieurs phases d'énumération des micro-organismes. Pour des applications particulières, telles que le contrôle microbiologique agroalimentaire, une phase de confirmation peut également être requise, afin de répondre aux normes en vigueur dans ce domaine.

La phase de détection se base historiquement sur la croissance (culture) des micro-organismes sur milieux essentiellement gélosés, et par la mise en évidence des caractères métaboliques des micro-organismes recherchés. On utilise classiquement des substrats enzymatiques spécifiques. Ces substrats peuvent être des composés utilisés dans le métabolisme bactérien et conduisant à une modification du milieu détectée par des indicateurs (variation de pH, réduction, précipitation, etc.). Dans d'autres cas, ces substrats enzymatiques sont composés de deux parties, une première partie spécifique de l'activité enzymatique à révéler, appelée également partie cible, et une seconde partie faisant office de marqueur, appelée partie marqueur, généralement constituée par un chromophore ou un fluorophore. Par le choix de ces substrats, selon qu'il y a réaction ou non, il est possible de caractériser la nature d'un micro-organisme ou de discriminer différents groupes de micro-organismes. Ainsi, l'apparition ou la disparition d'une coloration ou d'une fluorescence sera la signature d'un genre ou d'un type de micro-organismes. A cet égard, l'utilisation de milieux chromogènes permet la détection et l'identification simultanées des germes recherchés. Elle simplifie le processus et diminue sensiblement le délai d'obtention du résultat. On citera à titre d'exemple concret les milieux ChromlD^{®} de la demanderesse. Ces milieux chromogènes sont basés sur la détection de caractères métaboliques spécifiques des germes recherchés comme par exemple l'activité enzymatique beta-glucuronidase pour Escherichia coli.

Les immuno-essais constituent une autre des technologies utilisées pour le test de détection. Ils font appel aux caractéristiques immunogènes des micro-organismes recherchés. De façon non exhaustive, on peut citer les techniques ELISA (« Enzyme Linked Immuno Sorbent Assay »), par compétition ou de type sandwich.

Enfin, les techniques de biologie moléculaire, basées sur les caractères génomiques des micro-organismes recherchés, sont également mises en oeuvre pour détecter et identifier les micro-organismes cibles. Ces techniques de biologie moléculaire offrent des perspectives extrêmement intéressantes. On citera à titre d'exemple les techniques d'amplification classiques telles la PCR (« Polymerase Chain Reaction » en langue anglaise) et la NASBA (« Nucleic Acid Sequence Based Amplification » en langue anglaise), qui peuvent être couplées à des techniques de détection en temps réel connues de l'homme du métier.

La phase de confirmation, quant à elle, est parfois nécessaire pour confirmer la présence du pathogène recherché, lorsque le résultat des méthodes développées précédemment est positif. Ceci impose un test complémentaire et l'utilisation d'un principe de détection différent de celui utilisé lors de la première analyse. Les techniques décrites supra sont utilisées à loisir pour la confirmation.

L'identification complète et précise d'un micro-organisme dans un échantillon nécessite donc l'enchaînement de plusieurs étapes : enrichissement, détection et, le cas échéant, confirmation. La standardisation des tests utilisés en routine a permis l'automatisation des méthodes de détection qui restent cependant longues à mettre en oeuvre. Un inconvénient des méthodes traditionnelles d'analyse utilisées dans l'état de la technique réside dans le fait que les étapes d'enrichissement, de détection et, le cas échéant, de confirmation sont réalisées de manière séquentielle et nécessitent un grand nombre de manipulations chronophages, impactant ainsi le temps nécessaire au rendu des résultats.

Tel qu'indiqué précédemment, la phase de détection est généralement précédée d'au moins une phase de pré-enrichissement et/ou d'enrichissement (plus généralement dénommée phase ou étape d'enrichissement aux fins de la présente demande). Cette dernière est primordiale dans la mesure où, à l'heure actuelle, il n'existe pas de méthode pour détecter un micro-organisme cible dans un échantillon biologique, présent en quantité minime, par exemple de l'ordre de quelques cellules dans l'échantillon, sans faire appel à une étape préalable d'enrichissement. Cette phase d'enrichissement requiert l'utilisation de milieux de culture, sélectifs ou non (en fonction du but recherché), qui ont pour but de promouvoir la croissance des micro-organismes cibles dans les échantillons biologiques ou environnementaux, tout en limitant la croissance des flores non cibles. Les milieux de culture sont fréquemment utilisés dans des conteneurs de type sac en plastique stérile, dans lesquels ils sont mis en contact avec les échantillons alimentaires ou environnementaux, aux fins de remise en suspension et enrichissement des micro-organismes recherchés. Tel que mentionné supra, cette phase d'enrichissement est nécessaire notamment afin de révéler la présence d'au moins un micro-organisme cible dans une quantité d'échantillon très variable et éventuellement très grande, par exemple de 25 grammes (g) à 375 g dilués dans un volume de milieu de culture compris entre 225 et 3375 millilitres (mL). A l'issue de cette étape d'enrichissement, un aliquote (généralement d'un volume compris entre 5 microlitres (µL) et 5 mL) est traditionnellement prélevé pour mettre en oeuvre l'étape de détection des micro-organismes cibles. Or, dans cet aliquote, il est nécessaire de disposer d'une quantité suffisante de micro-organismes cibles pour s'assurer de leur détection systématique.

Ainsi en fin d'incubation et malgré l'emploi de milieux sélectifs, la concentration en micro-organismes cibles reste dans certains cas insuffisante et/ou la concentration en flore annexe demeure trop importante pour effectuer une détection efficace des micro-organismes cibles. Dans cette configuration, l'homme du métier a généralement recours à des méthodes de traitement de l'échantillon ayant pour objectif d'augmenter le rapport entre la concentration en micro-organismes cibles et la concentration en flore annexe. Par exemple, après enrichissement, une fraction de l'échantillon (préférentiellement entre 1 et 10 mL) est traitée par une méthode d'immuno-concentration en utilisant des billes magnétiques fonctionnalisées par des anticorps spécifiques des micro-organismes cibles.

Or cette méthode de traitement de l'échantillon est limitée par le faible volume de l'aliquote utilisé. Elle est fastidieuse car complètement manuelle et présente, en outre, un risque de contamination de l'utilisateur car l'échantillon, contenant potentiellement des agents pathogènes, est manipulé après enrichissement.

En outre, l'étape d'enrichissement requiert non seulement un milieu de culture ad hoc mais également une incubation de l'ensemble formé au moins par l'échantillon biologique et le milieu de culture à une température optimale pour permettre la croissance du/des micro-organisme(s) cible(s). L'incubation s'effectue, en général, à une température allant de 25 à 45°C durant un laps de temps prédéterminé (par exemple de 6h à 48h). Or, durant cette période d'incubation, aucune action supplémentaire n'est pratiquée sur l'échantillon. Ce laps de temps n'est donc pas mis à profit, il est en quelque sorte « perdu ». Or, cela va à l'encontre de la problématique présentée supra, visant à mettre au point une technique d'analyse précise et rapide. En effet, durant cette étape d'enrichissement, l'échantillon est immobilisé dans une étuve sans moyen d'intervention car cette étape s'effectue en général pendant la nuit.

Le document CA 2264206 décrit un dispositif comprenant une pluralité de chambres de sacs de réaction reliées entre elles et à une colonne de séparation pour la culture de cellules anti-tumorales.

Eu égard à la somme des problématiques développées supra, un des objectifs de la présente invention vise à améliorer la capture et/ou la concentration des micro-organismes cibles (d'intérêt) ou des protéines issues desdits microorganismes en augmentant le temps de contact avec un support de capture et/ou concentration.

Un autre objectif de la présente invention est de contrôler le volume d'échantillon en contact avec les partenaires de liaisons du support de capture, le temps de contact de l'échantillon avec ces partenaires de liaison et donc le débit de l'échantillon au travers le support de capture. Cette nouvelle technique permet ainsi d'optimiser les conditions de réactions des partenaires de liaisons avec les microorganismes ou les protéines issues de ces microorganismes contenu(e)s dans l'échantillon.

Un autre objectif de la présente invention vise à proposer un traitement de l'échantillon (de préférence de manière automatisée) sur un grand volume d'échantillon et non pas uniquement sur un aliquote, ledit traitement consistant à capturer et concentrer les micro-organismes cibles (d'intérêt) ou les protéines issues desdits microorganismes en vue d'améliorer la sensibilité et la spécificité de la méthode de détection utilisée post-traitement.

L'invention a également pour objectif de valoriser/optimiser le temps d'incubation durant la phase d'enrichissement de l'échantillon biologique en effectuant, pendant la période d'incubation/enrichissement et de croissance des micro-organismes, une capture et/ou une concentration des micro-organismes cibles présents dans l'échantillon (de préférence de manière automatisée).

Un autre objectif de la présente invention est de fournir un procédé permettant d'augmenter la cadence d'analyse des échantillons et/ou de diminuer le temps total nécessaire à l'analyse de l'échantillon biologique.

Un autre objectif de la présente invention vise à limiter les manipulations de l'échantillon contenu dans le conteneur, limitant de ce fait les risques de contamination, soit du personnel manipulant l'échantillon, soit de l'échantillon lui-même. A cet égard, la présente invention a également pour objet la mise au point d'un procédé de préparation d'un échantillon automatisé ou semi-automatisé.

Un autre objectif de la présente invention est d'améliorer la traçabilité de l'analyse du fait de la réduction drastique des étapes de manipulation des échantillons.

D'autres objectifs apparaîtront à la lecture de la présente demande.

La présente invention vise donc à atteindre tout ou partie des objectifs précités.

### Exposé de l'invention

Le conteneur utilisé aux fins de la présente invention comprend :
- un dispositif de capture et/ou concentration de microorganisme ou de protéine issue dudit microorganisme, comprenant un support de capture et/ou concentration, ledit support de capture étant une membrane poreuse,
- un récipient comprenant au moins deux parois dont au moins une est flexible, adapté pour recevoir au moins un échantillon susceptible de contenir au moins un microorganisme cible, ledit récipient comprenant
   o un orifice d'entrée emménagé au travers d'une desdites parois et permettant le passage de l'échantillon contenu dans le récipient vers le dispositif de capture et/ou concentration
   o un guide fluidique d'entrée, susceptible de contenir un volume d'échantillon défini et permettant de guider ledit volume d'échantillon vers ledit orifice d'entrée
   o au moins un moyen de contrôle du débit d'échantillon transféré du guide fluidique vers le dispositif de capture et/ou concentration au travers dudit orifice d'entrée
   o un orifice de sortie emménagé au travers d'une desdites parois permettant le passage de l'échantillon du dispositif de capture et/ou concentration vers le récipient,
ledit dispositif de capture et/ou concentration étant fixé à une paroi du récipient.

Selon la présente invention, le conteneur comprend un dispositif de capture et/ou concentration de microorganisme ou de protéine issue dudit microorganisme, comprenant un support de capture et/ou concentration. Avantageusement, le dispositif de capture et/ou concentration, est en partie accolé à la face extérieure d'une desdites parois du récipient. Encore préférentiellement, le récipient comprend au moins deux connecteurs étanches adaptés aux orifices d'entrée et de sortie du récipient. Les connecteurs étanches empêchent alors l'échantillon de se répandre hors du conteneur et ainsi de contaminer l'environnement ou le technicien manipulant l'échantillon.

Dans un mode de réalisation préféré, le dispositif de capture/concentration peut se présenter sous la forme d'une cartouche fixée au récipient. Préférentiellement, le dispositif de capture et/ou concentration peut être désolidarisé de la paroi dudit récipient.

Dans un mode particulier, le dispositif de capture/concentration est composé de deux parties :
- une première partie correspondant à un porte-cartouche fixable à la paroi du récipient
- et une deuxième partie correspondant à la cartouche comprenant le support de capture.

Selon ce mode particulier, la présente invention porte sur un conteneur tel que décrit précédemment caractérisé en ce que le dispositif de capture et/ou concentration de microorganisme ou de protéine issue dudit microorganisme comprend
- un porte-cartouche comprenant :
   - au moins un moyen d'insertion permettant à une cartouche d'être insérée dans le porte-cartouche,
   - au moins un premier conduit d'admission permettant l'entrée d'un échantillon liquide ; le premier conduit d'admission étant en liaison avec une voie d'admission de la cartouche lorsque le porte-cartouche et la cartouche sont positionnés selon un chemin fluidique dit de « capture/concentration »,
   - au moins un deuxième conduit d'admission permettant l'entrée d'un liquide de lavage et/ou d'un liquide de lyse et/ou un liquide d'élution ; le deuxième conduit d'admission étant en liaison avec une voie d'admission de la cartouche lorsque le porte-cartouche et la cartouche sont positionnés selon un chemin fluidique dit « de lyse et/ou lavage et/ou élution»,
   - au moins un conduit d'évacuation permettant la sortie de l'échantillon liquide et/ou du liquide de lavage, de lyse ou d'élution; le conduit d'évacuation étant en liaison avec la voie d'évacuation de la cartouche.
- une cartouche comprenant :
   - au moins un moyen d'insertion permettant à la cartouche d'être insérée dans au moins deux positions différentes, une première position selon un chemin fluidique dit de « capture/concentration » et une deuxième position selon un chemin fluidique dit de « lyse et/ou lavage et/ou élution»,
   - un module réactionnel comprenant
      - au moins une voie d'admission de l'échantillon liquide et/ou du liquide de lavage et/ou de lyse et/ou d'élution permettant son (leur) entrée à l'intérieur du module,
      - au moins une voie d'évacuation de l'échantillon liquide et/ou du liquide de lavage et/ou de lyse et/ou d'élution permettant son (leur) évacuation vers le porte-cartouche puis vers le conteneur,
      - un support de capture et/ou concentration disposé entre la voie d'admission et la voie d'évacuation de telle sorte que tout ou partie de l'échantillon ou des liquides le traverse; ledit support de capture et/ou concentration étant une membrane poreuse.

La cartouche peut comporter un couvercle et un fond assemblés par clipsage, collage, soudage, bouterollage, rivetage, sertissage, ou tout autre moyen permettant un assemblage fiable et résistant. L'étanchéité entre le couvercle et le fond de la cartouche peut être réalisée par simple collage. Elle peut être aussi réalisée par ajout d'une colle, silicone, mastic entre les faces ad-hoc du couvercle et du fond de la cartouche. Elle peut également être réalisée au moyen d'un joint et contre-joint.

Le support de capture est disposé à l'intérieur du module de réaction de la cartouche.

La cartouche et le porte-cartouche comprennent un moyen d'insertion. On entend par « moyen d'insertion » tout moyen permettant de guider la cartouche dans le porte-cartouche. Il peut, par exemple, s'agir de glissières. De façon préférentielle, le moyen d'insertion contient un détrompeur, par exemple une glissière asymétrique, permettant de guider la cartouche dans le porte-cartouche dans un seul sens.

Préférentiellement, le dispositif est fixé à une paroi d'un récipient adapté pour recevoir un échantillon. Préférentiellement, le porte-cartouche et/ou la cartouche peut (peuvent) être désolidarisé(e)(s) de la paroi dudit récipient.

Dans un mode particulier, une base est accolée à la face interne de la paroi du récipient et comprend
- au moins un canal d'admission de l'échantillon liquide en liaison fluidique avec la voie d'admission de l'échantillon de la cartouche via le premier conduit d'admission du porte-cartouche,
- au moins un canal d'évacuation de l'échantillon liquide et/ou de lavage et/ou de lyse, en liaison fluidique avec la voie d'évacuation de la cartouche via le conduit d'évacuation du porte-cartouche.

La cartouche, le porte-cartouche et la base peuvent être constitués d'un matériau plastique, par exemple ils peuvent être injectés dans un polymère de la classe des polyoléfines, ou les polyamides, ou des polyesters (non limitatives). Certains matériaux biosourcés peuvent aussi être envisagés.

Selon un mode de réalisation préférentiel, la base est connectée à un guide fluidique permettant à l'échantillon contenu dans le récipient d'arriver au canal d'admission de la base.

Le conteneur utilisé aux fins de la présente invention comprend un récipient ouvert ou clos (par exemple de manière hermétique ou étanche) comprenant au moins deux parois dont au moins une est flexible. Selon un mode de réalisation préféré, le récipient est un sac possédant une enveloppe flexible du type sac d'homogénéisation. De préférence, au moins une paroi du récipient est transparente afin d'être en mesure de percevoir le volume occupé par le liquide à l'intérieur du récipient.

Selon la présente invention, le récipient comprend un guide fluidique d'entrée, susceptible de contenir un volume d'échantillon défini et permettant de guider ledit volume d'échantillon vers ledit orifice d'entrée.

Le guide fluidique peut être constitué par tout moyen permettant de guider l'échantillon biologique contenu dans le récipient vers l'orifice d'entrée du dispositif de capture/concentration.

Selon un mode de réalisation particulièrement préféré le guide fluidique est élastique et destiné à être pressé. Il peut s'agir d'un tube en matière silicone par exemple.

Selon un autre mode de réalisation, le guide fluidique est une poche interne du récipient destinée à être pressée. Préférentiellement, au moins une partie de la poche interne est une partie de la paroi flexible du récipient.

Le guide fluidique peut être constitué par tout moyen permettant de guider l'échantillon biologique contenu dans le récipient au canal d'admission de la base. Selon un mode de réalisation particulièrement préféré le guide fluidique est élastique et destiné à être pressé. Il peut s'agir d'un tube en matière silicone par exemple.

Selon l'invention, le récipient peut comprendre un guide fluidique de sortie permettant une communication fluidique entre l'orifice de sortie du dispositif de capture et/ou concentration et le récipient.

Le guide fluidique peut éventuellement être muni d'un système d'évent empêchant que les parois du récipient ne se collent entre elles et obstruent le passage vers le dispositif de capture et/ou concentration.

Selon l'invention, le récipient comprend un moyen de contrôle du volume d'échantillon transféré du guide fluidique vers le dispositif de capture et/ou concentration au travers dudit orifice d'entrée. Dans un mode préféré, le moyen de contrôle du volume est une section d'un diamètre déterminé du guide fluidique et la taille (la longueur) du guide fluidique.

Selon un mode de réalisation de l'invention, le récipient peut comprendre un compartiment servant à recevoir le filtrat de l'échantillon, un filtre séparant ledit récipient en deux compartiments, l'un servant à recevoir l'échantillon, l'autre servant à recevoir l'échantillon filtré.

Selon un autre mode de réalisation, le guide fluidique comprend un filtre évitant que le support de capture ne soit obstrué avec certains échantillons.

Si l'on considère la partie supérieure du conteneur comme étant la partie « haute » et la partie inférieure de ce conteneur comme étant la partie « basse », l'orifice d'entrée peut être située dans la partie basse et l'orifice de sortie dans la partie haute. Dans ce cas-là, en schématisant, la circulation de l'échantillon à travers le support de capture/concentration se fait de bas en haut. Dans un autre mode de réalisation, l'orifice d'entrée peut être située dans la partie haute et l'orifice de sortie dans la partie basse. Dans ce cas-là, la circulation de l'échantillon à travers le support de capture/concentration se fera par gravitation de haut en bas.

Dans le mode de réalisation particulier du conteneur comprenant une cartouche et un porte-cartouche, si l'on considère la partie supérieure du conteneur comme étant la partie « haute » et la partie inférieure de ce conteneur comme étant la partie « basse », la voie d'admission et le premier conduit d'admission de l'échantillon peuvent être situés dans la partie basse et la voie d'évacuation et le conduit d'évacuation dans la partie haute. Dans ce cas-là, en schématisant, la circulation de l'échantillon à travers le support de capture/concentration se fait de bas en haut. Dans un autre mode de réalisation, la voie d'admission et le premier conduit d'admission peuvent être situés dans la partie haute et la voie et le conduit d'évacuation dans la partie basse. Dans ce cas-là, la circulation de l'échantillon à travers le support de capture/concentration se fera par gravité de haut en bas.

Dans un mode de réalisation particulier, le dispositif de capture et/ou concentration comprend un moyen de récupération des acides nucléiques une fois que les microorganismes aient été lysés par le liquide de lyse, apte à être placé en communication fluidique avec le deuxième conduit d'admission du porte-cartouche.

Selon la présente invention, l'échantillon peut être de diverses origines, par exemple d'origine alimentaire, environnementale, vétérinaire ou clinique. Parmi les échantillons d'origine alimentaire, l'on peut citer, de façon non-exhaustive, un échantillon de produits lactés (yaourts, fromages...), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non (ou partiellement) transformées. Un échantillon alimentaire peut enfin être issu d'une alimentation destinée aux animaux, telle que des tourteaux, des farines animales. A titre d'exemple d'échantillons, il convient également de mentionner les échantillons liés à l'environnement tels les prélèvements de surface, d'eau, d'air, etc.

Les échantillons d'origine clinique peuvent correspondre à des prélèvements de fluides biologiques (sang total, sérum, plasma, urine, liquide céphalo-rachidien, etc.), de selles, de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité (plus particulièrement une petite partie ou une petite quantité) prélevées à partir d'une ou plusieurs entités aux fins d'analyse. Cet échantillon peut éventuellement avoir subi un traitement préalable, impliquant par exemple des étapes de mélange, de dilution, encore de broyage, en particulier si l'entité de départ est à l'état solide, ou encore de pré-enrichissement ou d'enrichissement par mise en contact avec un milieu de culture. Il peut également être filtré préalablement ou au sein même du récipient ou du dispositif de capture/concentration. L'échantillon est donc sous forme liquide au cours du procédé selon la présente invention.

L'échantillon outre le milieu de culture peut, bien évidemment, comprendre des éléments additionnels, tels que des vitamines ou autres éléments nutritifs utiles à la culture de micro-organismes, des agents sélectifs, des substrats spécifiques et autres éléments bien connus de l'homme du métier.

L'échantillon analysé est, en général, susceptible de - ou suspecté de - contenir au moins un micro-organisme cible. Dans la plupart des cas, ce dernier est un micro-organisme pathogène (tel que Salmonella) qu'il convient de détecter à des fins sanitaires.

Le terme « micro-organisme » a la même signification que celle généralement admise en microbiologie et comprend notamment les bactéries gram positif ou gram négatif, les levures, les moisissures et plus généralement, les organismes unicellulaires, invisibles à l'oeil nu, qui peuvent être manipulés et multipliés en laboratoire.

Selon un mode de réalisation préféré, le ou les micro-organisme(s) à détecter sont des bactéries, par exemple des entérobactéries telles que E. Coli.

La présente invention s'applique également à la capture et/ou concentration de protéine(s) issue(s) dudit microorganisme telle que les toxines. A titre illustratif, l'on peut citer la détection des toxines sécrétées par Staphylococcus aureus.

Avantageusement, l'échantillon comprend au moins un milieu de culture permettant la croissance des micro-organismes et, en particulier du ou des micro-organismes cibles. Par « milieu de culture », l'on entend un milieu comprenant tous les éléments nécessaires à la survie et/ou à la croissance des micro-organismes et, en particulier des micro-organismes recherchés (par exemple de l'eau tamponnée peptonée). Le milieu de culture peut contenir d'éventuels additifs, par exemple : des peptones, un ou plusieurs facteurs de croissance, des hydrates de carbone, un ou plusieurs agents sélectifs, des tampons, un ou plusieurs gélifiants, une ou plusieurs vitamines, etc. Ce milieu de culture peut se présenter sous forme liquide ou gélifiée prête à l'emploi, à savoir prête à être ensemencée en tube, en flacon ou sur boîte de Pétri. L'expression « milieu de culture » englobe bien évidemment les milieux et bouillons d'enrichissement.

Par «support de capture et/ou concentration», l'on entend tout support permettant de fixer le microorganisme ou la protéine dudit microorganisme recherché(e) et faire en sorte qu'il(elle) soit en concentration suffisante aux fins des étapes ultérieures d'analyse. Le «support de capture et/ou concentration» sera également mentionné indifféremment par «support de capture/concentration».

Le support de capture et/ou concentration est un support de capture et/ou de concentration poreux. Il s'agit d'une membrane poreuse.

Avantageusement, le support de capture et/ou concentration est fonctionnalisé avec au moins un partenaire de liaison spécifique du micro-organisme cible ou de protéine issue dudit microorganisme.

Selon un mode de réalisation préféré, le partenaire de liaison spécifique est sélectionné dans le groupe comprenant les anticorps, les fragments Fab, les fragments Fab', les protéines de phages recombinantes ou non, les phages ou tout autre ligand bien connu de l'homme du métier. L'analyse peut être réalisée in situ à partir du support de capture et concentration. La révélation de la présence des micro-organismes cibles au niveau du support de capture et concentration peut être effectuée par l'intermédiaire de tout système de révélation approprié, c'est-à-dire apte à permettre la détection du ou des micro-organisme(s) cible(s). Par « système de révélation », l'on entend toute molécule capable de se coupler avec les micro-organismes ou les partenaires de liaison desdits micro-organismes en permettant, par leurs propriétés de transduction (fluorescence, coloration, radioactivité, etc.) de révéler la présence desdits micro-organismes. Cette révélation de la présence des micro-organismes cibles peut être notamment obtenue par visualisation (à l'oeil nu) ou lecture optique (via un dispositif de lecture optique de type caméra) d'une coloration (telle qu'une coloration rouge due à la réduction du TTC en formazan par les micro-organismes) ou d'une fluorescence sur tout ou partie du support de capture.

De manière non-limitative, l'analyse peut être réalisée par tout moyen de détection, ledit moyen de détection pouvant être sélectionné parmi les moyens de détection optiques, les moyens de détection électriques (notamment électrochimiques), les moyens de détection acoustiques, les moyens de détection thermiques, les moyens de détection mécaniques, les moyens de détection magnétiques.

Dans un mode de réalisation particulier, le conteneur peut être disposé dans un dispositif comprenant au moins un emplacement pour recevoir ledit conteneur, ledit dispositif comprenant
- au moins un moyen de pression sur le guide fluidique afin d'emprisonner dans le guide fluidique un volume déterminé d'échantillon
- et au moins un moyen de déplacement pour générer le déplacement de l'échantillon contenu dans le récipient vers le guide fluidique et/ou du guide fluidique vers le support de capture/concentration.

Ainsi, le déplacement de l'échantillon peut être exercé manuellement ou mécaniquement. Un tel dispositif est décrit dans la demande de brevet WO2014072438 déposée par la demanderesse. Il peut s'agir d'un dispositif à pales (ou à bras), lesdites pales venant comprimer le récipient à paroi flexible (par exemple en matière plastique de type PVC, polyéthylène, polyester). Ce dispositif permet une maîtrise optimale du débit de circulation de l'échantillon dans le support de capture/concentration.

Le moyen de pression peut être, quant à lui, tout moyen permettant de pincer le guide fluidique, empêchant à l'échantillon à l'extérieur du guide fluidique de rentrer à l'intérieur du guide fluidique.

La présente invention a également pour objet la mise au point d'un procédé de préparation d'un échantillon susceptible de contenir au moins un micro-organisme cible comprenant essentiellement les étapes suivantes :
a) disposer ledit échantillon dans un conteneur comprenant
   - un dispositif de capture et/ou concentration de microorganisme cible ou de protéine issue dudit microorganisme, comprenant un support de capture et/ou concentration, ledit support de capture étant une membrane poreuse;
   - un récipient avec au moins deux parois dont au moins une est flexible, adapté pour recevoir ledit échantillon susceptible de contenir au moins un microorganisme cible, ledit récipient comprenant
      o un orifice d'entrée emménagé au travers d'une desdites parois et permettant le passage de l'échantillon liquide contenu dans le récipient vers le dispositif de capture et/ou concentration
      o un guide fluidique d'entrée, susceptible de contenir un volume d'échantillon défini et permettant de guider ledit volume d'échantillon vers ledit orifice d'entrée
      o au moins un moyen de contrôle du volume d'échantillon transféré du guide fluidique vers le dispositif de capture et/ou concentration au travers dudit orifice d'entrée
      o un orifice de sortie emménagé au travers d'une desdites parois permettant le passage de l'échantillon du dispositif de capture et/ou concentration vers le récipient,
   ledit dispositif de capture/concentration était fixé à une paroi du récipient.
b) générer un déplacement de tout ou partie de l'échantillon vers le guide fluidique,
c) générer un déplacement d'un volume défini de l'échantillon du guide fluidique vers le dispositif de capture et/ou concentration au travers l'orifice d'entrée,
d) capturer et/ou concentrer sur le support de capture et/ou concentration ledit microorganisme cible ou une protéine issue dudit microrganisme cible contenu(e) dans l'échantillon, ledit volume d'échantillon traversant le support de capture et/ou concentration puis ressortant à travers l'orifice de sortie du récipient.
e) répéter la circulation de l'échantillon en flux tangentiel à travers le support de capture.

La présente invention a également pour objet la mise au point d'un procédé de préparation d'un échantillon susceptible de contenir au moins un micro-organisme cible tel que décrit supra caractérisé en ce que le dispositif de capture et/ou concentration comprend
- un porte-cartouche comprenant :
   - au moins un moyen d'insertion permettant à une cartouche d'être insérée dans le porte-cartouche,
   - au moins un premier conduit d'admission permettant l'entrée d'un échantillon liquide; le premier conduit d'admission étant en liaison avec la voie d'admission de la cartouche lorsque le porte-cartouche et la cartouche sont positionnés selon un chemin fluidique dit de « capture/concentration »,
   - au moins un deuxième conduit d'admission permettant l'entrée d'un liquide de lavage et/ou d'un liquide de lyse et/ou un liquide d'élution ; le deuxième conduit d'admission étant en liaison avec une voie d'admission de la cartouche lorsque le porte-cartouche et la cartouche sont positionnés selon un chemin fluidique dit « de lyse et/ou lavage et/ou élution».
   - au moins un conduit d'évacuation permettant la sortie de l'échantillon liquide et/ou du liquide de lavage ou de lyse vers le conteneur; le conduit d'évacuation étant en liaison avec la voie d'évacuation de la cartouche.
- une cartouche comprenant :
   - au moins un moyen d'insertion permettant à la cartouche d'être insérée dans au moins deux positions différentes, une première position selon un chemin fluidique dit de « capture/concentration » et une deuxième position selon un chemin fluidique dit de « lyse et/ou lavage et/ou élution».
   - un module réactionnel comprenant
      - au moins une voie d'admission de l'échantillon liquide et/ou du liquide de lavage et/ou de lyse et/ou d'élution permettant son (leur) entrée à l'intérieur du module,
      - au moins une voie d'évacuation de l'échantillon liquide et/ou du liquide de lavage et/ou de lyse et/ou d'élution permettant son (leur) évacuation vers le porte-cartouche puis vers le conteneur,
      - un support de capture et/ou concentration disposé entre la voie d'admission et la voie d'évacuation de telle sorte que tout ou partie de l'échantillon ou des liquides le traverse.

De préférence, le déplacement de l'échantillon vers le dispositif de capture et/ou concentration est effectué mécaniquement et de façon répétée.

La recirculation de l'échantillon à travers le support de capture permet d'augmenter le temps de contact de microorganisme avec le support de capture/concentration et donc d'augmenter la probabilité de rencontre du microorganisme avec le support de capture/concentration.

Selon la présente invention, l'échantillon peut éventuellement avoir subi un traitement préalable, impliquant par exemple des étapes de pré-enrichissement ou d'enrichissement par mise en contact avec un milieu de culture. Cette étape de mise en contact de l'échantillon avec un milieu de culture peut se faire également au sein du conteneur selon l'invention.

Ainsi, avantageusement, ledit procédé comprend une étape d'incubation dudit échantillon à une température et durant un laps de temps suffisant pour permettre la croissance dudit au moins un micro-organisme cible, l'étape d'incubation ayant lieu avant l'étape a) ou pendant tout ou partie des étapes a) à d). Dans ce contexte, le temps d'incubation peut être optimisé en choisissant d'effectuer la capture/concentration pendant l'incubation.

Concernant l'étape d'incubation, l'homme du métier saura, à partir de son expérience, de ses connaissances générales et/ou des données bibliographiques à sa disposition, adapter la température et le laps de temps nécessaires pour permettre une croissance suffisante du micro-organisme cible, en fonction du type de micro-organisme recherché. A titre informatif, et tel que mentionné dans le préambule de la présente demande, l'incubation s'effectue, en général, à une température allant de 25 à 45°C durant un laps de temps prédéterminé (par exemple de 6h à 48h).

Le déplacement de tout ou partie de l'échantillon vers le guide fluidique puis vers le dispositif de capture et/ou concentration au travers l'orifice d'entrée peut être obtenu par tout moyen connu par l'homme du métier. Le(s) guide(s) fluidique(s) permet(tent) au contenu de rentrer en contact avec l'orifice d'entrée tout en empêchant au contenu de rentrer en contact avec l'orifice de sortie s'il n'est pas passé à travers le dispositif de capture et concentration.

En particulier, ce déplacement peut être généré par l'application d'une force ou d'un ensemble de forces sur le conteneur ou encore au changement du bilan des forces s'appliquant au conteneur. Ainsi, par exemple, si le conteneur est tenu en deux points par deux forces de maintien et si l'une des deux forces cesse, le conteneur va s'incliner et le niveau du contenu va varier, au niveau d'au moins une partie de la surface interne du conteneur, du niveau de repos vers un niveau situé au-dessus de ce dernier et va possiblement être mis en contact avec le moyen destiné à créer l'orifice d'entrée via le guide fluidique. Le déplacement du contenu peut également être obtenu en appliquant une force ou un système de forces à l'intérieur du récipient, par exemple en gonflant et dégonflant une poche flexible gonflable (telle qu'un ballon ou un boudin gonflable) placée dans l'enceinte du récipient. Avantageusement les étapes b) et c) se font à l'aide d'un dispositif comprenant un moyen de déplacement et un moyen de pression tel que décrit plus-haut, afin d'exercer une pression sur le guide fluidique et permettre qu'un volume déterminé de l'échantillon soit mis en contact avec le support de capture et/ou concentration. Un tel dispositif est décrit dans la demande de brevet WO2014072438 déposée par la demanderesse. Il peut s'agir d'un dispositif à pales (ou à bras), lesdites pales venant comprimer le récipient à paroi flexible (par exemple en matière plastique de type PVC, polyéthylène, polyester).

Selon l'invention, le support de capture et/ou concentration est une membrane poreuse. La circulation se fait en flux tangentiel au travers la membrane, ce qui a pour avantage d'augmenter le temps de contact entre le microorganisme et le support de capture. Avantageusement, tout ou partie de l'échantillon contenu dans le récipient circule à travers le support de capture/concentration à un débit compris entre 0.5 mL/min par cm² de support à 5 mL/min par cm² de support, ledit support étant poreux et ayant une surface développée d'au moins 20 cm².

La présente invention a pour avantage de permettre la capture puis la révélation de la présence de micro-organisme cible dans une quantité d'échantillon très variable et éventuellement très grande, par exemple de 25 grammes (g) à 375 g dilués dans un volume de milieu de culture compris entre 225 et 3375 mL. Ainsi le volume de l'échantillon qui peut passer à travers le dispositif de capture/concentration est compris entre 10 mL et 5000 mL, préférentiellement entre 50 mL et 4000 mL, plus préférentiellement entre 200 et 3500 mL, encore plus préférentiellement entre 225 et 3375 mL.

Ce procédé permet avantageusement d'utiliser un grand volume de départ pour effectuer la capture/concentration, ce qui permet d'augmenter la probabilité de rencontre du microorganisme cible (ou la protéine issue de ce microorganisme) avec le support de capture/concentration. Préférentiellement, au moins 10 mL de l'échantillon contenu dans le récipient circule à travers le support de capture et/ou concentration, préférentiellement au moins 50 mL.

Ainsi le procédé de préparation d'un échantillon, selon l'invention est un procédé de capture/concentration d'au moins un micro-organisme cible d'un échantillon susceptible de contenir ledit au moins un micro-organisme cible. Il s'agit d'un procédé destiné à permettre la capture/concentration d'au moins un micro-organisme cible, de préférence, en présence d'au moins un milieu de culture/bouillon d'enrichissement. Lorsque l'on soupçonne que le(s) micro-organisme(s) recherché(s) est/sont présent(s) en quantité minime, par exemple de l'ordre de quelques cellules, dans l'échantillon, ce procédé permet que ledit au moins un micro-organisme cible soit présent en fin de procédé selon l'invention à une concentration telle que l'utilisateur puisse éventuellement le détecter de manière systématique ou quasi-systématique en recourant aux méthodes traditionnelles de détection (culture sur milieux gélosés, immuno-essais, techniques de biologie moléculaire, etc.).

La présente invention a donc pour objet la mise au point d'un procédé de préparation d'un échantillon susceptible de contenir au moins un micro-organisme cible comprenant les étapes suivantes :
a) disposer ledit échantillon dans un conteneur comprenant un porte-cartouche et une cartouche tel que décrit ci-dessus, ledit porte-cartouche et ladite cartouche étant positionnés selon un chemin fluidique dit de « capture/concentration »,
b) générer un déplacement de tout ou partie de l'échantillon vers le guide fluidique,
c) générer un déplacement d'un volume défini de l'échantillon du guide fluidique vers le dispositif de capture et/ou concentration au travers l'orifice d'entrée,
d) capturer et/ou concentrer sur le support de capture et/ou concentration ledit microorganisme cible ou une protéine issue dudit microrganisme cible contenu(e) dans l'échantillon, ledit volume d'échantillon traversant le support de capture et/ou concentration puis ressortant à travers l'orifice de sortie du récipient.

Dans un mode de réalisation particulier, le procédé de préparation d'un échantillon selon la présente invention comprend également une ou plusieurs étape(s) de lavage par un liquide de lavage dispensé dans le deuxième conduit d'admission du porte-cartouche, la cartouche étant disposée selon un chemin fluidique dit de lyse et/ou lavage et/ou élution. Un mouvement de translation de la cartouche dans le porte-cartouche en suivant les glissières permet de passer d'un chemin fluidique dit de « capture/concentration » à un chemin fluidique dit de « lyse et/ou lavage et/ou élution».

Dans un autre mode de réalisation particulier, le procédé de préparation d'un échantillon selon la présente invention comprend également une ou plusieurs étape(s) de lyse par un liquide de lyse dispensé dans le deuxième conduit d'admission du porte-cartouche, la cartouche étant disposée selon un chemin fluidique dit de lyse et/ou lavage et/ou élution. Dans un autre mode de réalisation particulier, le procédé préparation d'un échantillon selon la présente invention comprend une ou plusieurs étape(s) d'élution par un liquide d'élution dispensé dans le deuxième conduit d'admission du porte-cartouche, ladite cartouche étant disposée selon un chemin fluidique dit de lyse et/ou lavage et/ou élution. Dans un autre mode de réalisation particulier, le procédé préparation d'un échantillon selon la présente invention comprend une étape ultérieure d'aspirer le liquide d'intérêt contenant les acides nucléiques desdits microorganismes libérés lors de la lyse.

Cette étape d'aspiration peut se faire à l'aide d'une seringue (celle-ci pouvant servir à l'injection de la solution de lavage/lyse) ou encore d'une pipette, d'un vacutainer^{®}, d'une psipette ou encore d'une pompe peristatique, mis en connexion avec le deuxième conduit d'admission du porte-cartouche.

Dans un autre mode de réalisation, le procédé de préparation comprend une étape ultérieure de décontamination par un décontaminant dispensé dans le deuxième conduit d'admission du porte-cartouche, la cartouche étant disposée selon un chemin fluidique dit de lyse et/ou lavage et/ou élution.

Avantageusement le procédé selon l'invention permet de capturer et concentrer efficacement un microorganisme cible d'un échantillon biologique tout en limitant les manipulations de l'échantillon contenu dans le récipient, limitant de ce fait les risques de contamination, soit du personnel manipulant l'échantillon, soit de l'échantillon lui-même.

Selon un mode de réalisation, le procédé selon l'invention comprend une étape ultérieure d'analyse dudit support de capture et concentration par lecture visuelle, optique ou tout autre moyen. Ainsi ce procédé permet d'analyser ledit ou lesdits micro-organisme(s) cible(s) et/ou tout ou partie de leurs propriétés. L'analyse peut notamment consister en un procédé de détection directe - et le cas échéant d'identification - dudit ou desdits micro-organisme(s) ou en un procédé de détection - et le cas échéant d'identification - indirecte, par exemple lié à la détection d'informations nucléotidiques et/ou protéiques spécifiques d'un type de micro-organisme à détecter et/ou identifier. Cette détection et/ou identification indirecte peut également résulter de la détection de protéines se liant spécifiquement à des protéines de bactériophages spécifiques dudit ou desdits micro-organisme(s) à détecter. La présence d'un micro-organisme cible peut également être décelée par une résistance à un antibiotique donné ou à un ensemble d'antibiotiques, le profil de résistance à cet ou ces antibiotique(s) étant, dans ce cas, caractéristique du ou des micro-organisme(s) à détecter. La détection directe ou indirecte peut se faire in situ sur le support de capture après par exemple ajout d'un système de révélation.

Dans un mode de réalisation particulier, l'analyse dudit support de capture et concentration se fait après désolidarisation du dispositif de capture/concentration et/ou dudit support de capture/concentration du récipient.

Ainsi, l'analyse du support de capture /concentration peut également être effectuée ex situ en transférant le dispositif et/ou le support de capture/concentration dans un dispositif d'analyse. Dans un mode de réalisation préféré, les microorganismes couplés au support de capture / concentration sont lysés afin de récupérer les acides nucléiques puis analysés par une méthode d'amplification telle que la PCR.

Le procédé selon la présente invention permet également d'effectuer des contrôles de stérilité notamment au sein des prélèvements alimentaires et environnementaux. Pour ce faire, l'on utilise, en tant que moyens d'analyse, des moyens de détection génériques de micro-organismes tels que des supports de capture fonctionnalisés avec des partenaires de liaison génériques de type anti-Gram-, anti-Gram+, etc. Le type d'analyse réalisé avec le procédé de l'invention peut donc être non seulement qualitatif (détection et identification de micro-organisme(s) spécifique(s)) mais également quantitatif ou semi-quantitatif.

### Brève description des dessins

L'invention, sa fonctionnalité, ses applications ainsi que ses avantages seront mieux appréhendés à la lecture de la présente description, faite en référence aux figures, dans lesquelles :
- la figure 1 représente de manière schématique un conteneur selon l'invention, le dispositif de capture/concentration étant à l'extérieur du récipient.
- la figure 2 représente de manière schématique un dispositif avec un conteneur, un moyen de pression sur le guide fluidique afin d'emprisonner dans le guide fluidique un volume déterminé d'échantillon, et un moyen de déplacement pour générer le déplacement de l'échantillon contenu dans le guide fluidique vers le dispositif de capture et/ou concentration.
- les figures 3, 4 et 5 visent à illustrer le dispositif tel que représenté figure 2 au cours des différentes étapes de mise en contact du moyen de déplacement et du moyen de pression avec le conteneur générant ainsi le déplacement de l'échantillon contenu dans le guide fluidique vers le dispositif de capture et/ou concentration.
- La figure 6 représente une cartouche selon l'invention, la cartouche étant ouverte.
- La figure 7 illustre une cartouche selon l'invention, la cartouche étant refermée.
- La figure 8 représente un porte-cartouche selon l'invention.
- La figure 9 montre une base et un guide fluidique selon l'invention.
- La figure 10 représente une cartouche insérée dans un porte-cartouche selon un chemin fluidique dit de « capture/concentration », le premier conduit d'admission du porte-cartouche étant en liaison fluidique avec la voie d'admission de la cartouche, le conduit d'évacuation du porte-cartouche étant en liaison avec la voie d'évacuation de la cartouche permettant ainsi à l'échantillon liquide de rentrer dans le module réactionnel et d'en ressortir.
- La figure 11 représente une cartouche insérée dans un porte-cartouche selon un chemin fluidique dit « de lyse et/ou lavage et/ou élution», le deuxième conduit d'admission du porte-cartouche étant en liaison fluidique avec la voie d'admission de la cartouche, le conduit d'évacuation du porte-cartouche étant en liaison avec la voie d'évacuation de la cartouche permettant ainsi au liquide de lyse et/ou lavage de rentrer dans le module réactionnel et d'en ressortir.
- La figure 12 est une illustration schématique d'un porte-cartouche fixé sur la paroi externe d'un récipient.

### Description détaillée du conteneur et du procédé selon l'invention

La description détaillée ci-après a pour but d'exposer l'invention de manière suffisamment claire et complète, notamment à l'aide d'exemples et de références à des figures, mais ne doit en aucun cas être regardée comme limitant l'étendue de la protection aux modes de réalisation particuliers objet desdits exemples et figures.

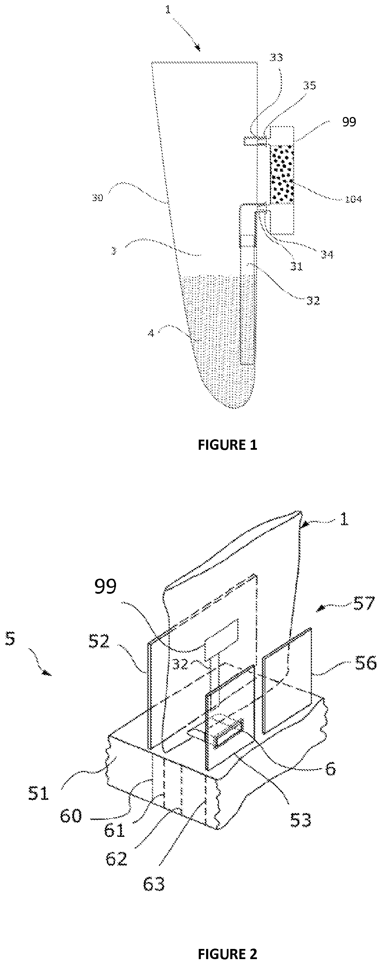

Le dispositif de préparation d'un échantillon (1), tel que représenté à la **figure 1**, conformément à un premier mode de réalisation, représente un récipient (3) comportant un dispositif de capture/concentration (99) fixé à la paroi externe qui peut être désolidarisé.

Ce récipient (3) comporte une paroi (30) souple, éventuellement transparente, à travers laquelle ont été emménagés un orifice d'entrée (31) et un orifice de sortie (33).

A l'intérieur du récipient un guide fluidique d'entrée (32) permet de guider l'échantillon (4) du récipient vers l'orifice d'entrée (31) puis vers le dispositif de capture et/ou concentration (99).

L'échantillon traverse le support de capture/concentration (104) puis passe à travers l'orifice de sortie (33) emménagé au travers de la paroi (30) souple vers l'intérieur du récipient.

Le dispositif de capture et/ou concentration comprend au moins deux connecteurs (34) et (35) adaptés aux orifices d'entrée et de sortie du récipient. Ces deux connecteurs sont résistants à la pression et adaptés de façon étanche empêchant l'échantillon de se répandre hors du conteneur et ainsi de contaminer l'environnement ou le technicien manipulant l'échantillon.

**La** **figure 2** représente un dispositif (5) comprenant un conteneur (1) positionné au sein du dispositif (5), dans un emplacement (57) prévu à cet effet.

Cet emplacement (57) est délimité d'un côté par la paroi fixe (52) et de l'autre par des moyens de déplacement représentés par un premier et un deuxième bras amovibles (semblables à deux pales) (53) et (56). Un des rôles des bras est d'homogénéiser, de malaxer l'échantillon. Les bras peuvent se déplacer à contre-sens et/ou en opposition de phase (alternance de phase).

Un moyen de pression (6) est associé à une des pales afin d'exercer une pression sur le guide fluidique (32), à travers la paroi souple du récipient. Le récipient et le guide fluidique viennent en butée contre la paroi fixe. Le moyen de pression en écrasant le guide fluidique à l'extrémité distale de l'orifice d'entrée contre la paroi fixe, emprisonne dans le guide fluidique un volume déterminé d'échantillon. Le guide fluidique est un tube élastique. Il peut s'agir d'un tube en silicone de faible épaisseur pour réduire la force d'écrasement mais avec des propriété élastique lui permettant un retour à sa géométrie initiale. Le moyen de contrôle du volume correspond au diamètre et à la longueur du tube. Le diamètre et la longueur du tube doivent être définis dans le but d'avoir un volume d'échantillon optimisé passant au travers du support de capture. Le diamètre et la longueur du tube seront également définis en fonction de la pression exercée sur le tube afin d'établir un débit optimisé au travers le support de capture.

Les moyens de déplacement (53) et (56) génèrent également le déplacement de l'échantillon contenu dans le guide fluidique vers le dispositif de capture et/ou concentration. Il convient de noter que le moyen de pression (6) peut être remplacés par des applicateurs de formes variées, pourvu que ceux-ci soient capables d'exercer une certaine force permettant de presser le guide fluidique contre la paroi fixe, la communication fluidique de l'échantillon de l'extérieur du guide vers l'intérieur du guide est ainsi bloquée. Le moyen de pression est une protubérance fixée à la pale (53).

A des fins de clarté, la figure 2 représente uniquement une partie d'un dispositif selon l'invention. Le dispositif (5) est pourvu d'une base (51) sur laquelle une paroi (52) a été fixée. Les deux pales (53) et (56), sont amovibles par rapport à la base (51). Le mouvement desdits bras (53) et (56) par rapport à la base (51) peut être généré par tout moyen adapté, tel qu'un moteur électrique.

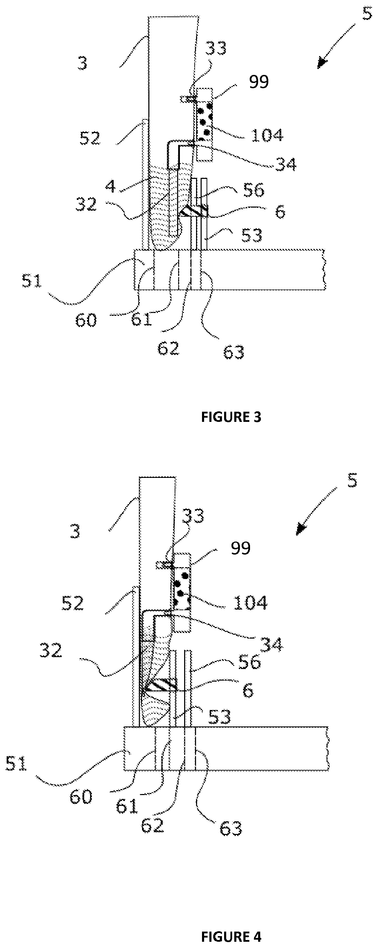

**Les** **figures 3 à 5** représentent le dispositif (5) en cours de fonctionnement à différentes étapes.

Dans **la** **figure 3**, le bras (53) est dans une position éloignée du conteneur, indiquée à l'aide de la ligne (63) et le bras (56) est dans une position plus rapprochée indiquée à l'aide de la ligne (62). La distance entre le bras (53) et le conteneur ne permet pas au moyen de pression (6) de presser le guide fluidique.

Dans la **figure 4**, le bras (53) passe à une position rapprochée du conteneur indiquée à l'aide de la ligne (61), la force exercée par le moyen de pression (6) sur le guide fluidique (32) en appuie sur la paroi fixe (52) entraine le pincement du guide fluidique, emprisonnant un volume d'échantillon en son sein.

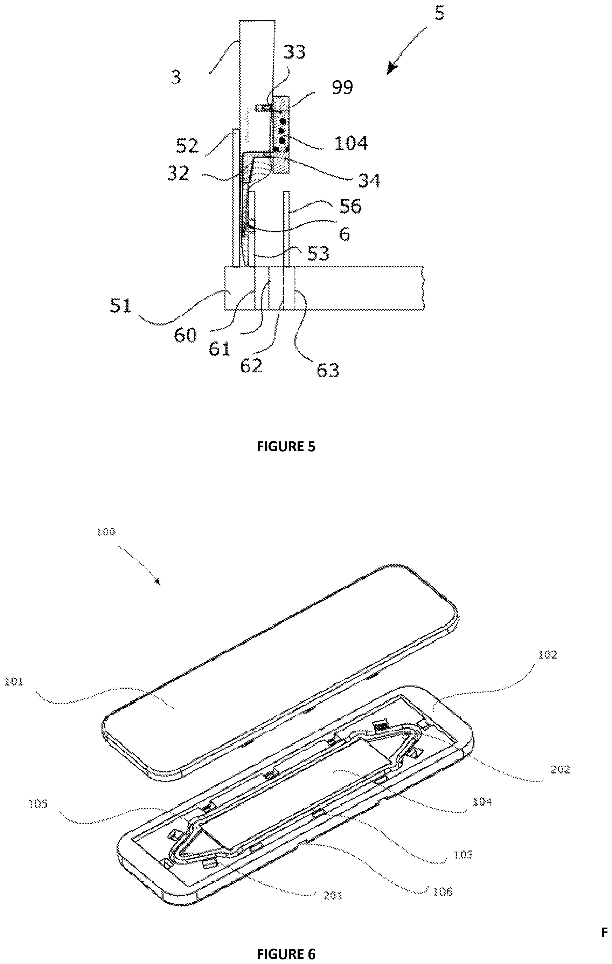

Dans la **figure 5**, le bras (53) passe à une position encore plus rapprochée du conteneur indiquée à l'aide de la ligne (60), le moyen de pression (6) se rétracte et le bras (53) en contact avec le conteneur entraine le déplacement du volume d'échantillon contenu dans le guide fluidique vers le dispositif de capture et/ou concentration.

Ainsi le dispositif de capture et/ou concentration est mis en contact avec les micro-organismes issus de l'échantillon biologique à analyser et, si les bactéries cibles sont présentes parmi lesdits micro-organismes, ces dernières se lient à leur partenaire spécifique de liaison présent au niveau dudit dispositif de capture et/ou concentration, par exemple à un anticorps fonctionnalisé. Les bactéries cibles sont alors (immuno)concentrées au niveau du dispositif de capture et/ou concentration et peuvent être identifiées in situ, par exemple par des techniques d'immuno-détection bien connues de l'homme du métier, mettant en oeuvre des systèmes de révélation également bien connus de celui-ci. Selon une variante, l'étape d'identification est réalisée à l'extérieur du conteneur, par exemple en mettant en oeuvre un automate de type VIDAS^{®}.

L'échantillon biologique (4) peut être tout échantillon pour lequel l'utilisateur souhaite contrôler la présence de micro-organismes d'intérêt. Tel qu'indiqué précédemment, l'échantillon peut être d'origine alimentaire, environnementale ou clinique (liste non-exhaustive) et les micro-organismes recherchés peuvent être des micro-organismes pathogènes, par exemple du type Salmonella ou E. Coli.

Lorsque le conteneur (1) est positionné dans l'emplacement (57), l'ensemble formé par le dispositif (5) et le conteneur (1) peut, par exemple, être mis en incubation à l'intérieur d'un incubateur (non représenté). Les conditions peuvent être optimisées à l'intérieur dudit incubateur pour permettre la croissance des micro-organismes recherchés. Des caractéristiques, telles que la température, peuvent être réglées afin d'être optimales pour favoriser la croissance des micro-organismes cibles. Lorsque l'ensemble formé du dispositif (5) et du conteneur (1), positionné dans l'emplacement (57), est introduit dans l'incubateur, le fonctionnement des bras (53) et (56) peut être activé.

Les bras (53) et (56) peuvent se déplacer, de leur première position (63) vers leur deuxième position (62) (et vice versa). Ce mouvement permet d'exercer une force sur la surface extérieure de la paroi souple du conteneur (1) et imposer ainsi audit conteneur (1) une déformation de cette paroi souple. A ce stade, les bras (53) et (56) peuvent servir à homogénéiser le contenu du conteneur (1).

Les bras (53) et (56) peuvent être déplacés de manière périodique. La fréquence peut être choisie et adaptée au type d'échantillon et/ou au type de milieu de culture présents à l'intérieur du conteneur (1).

A un moment choisi au cours de l'incubation ou après l'incubation, le bras sur lequel est fixé le moyen de pression (6) peut passer dans une position plus rapprochée, comme expliqué ci-dessus. En effet, un mode de réalisation avantageux est de différer la mise en contact de l'échantillon avec le dispositif de capture et/ou concentration sans pour autant attendre la fin de l'incubation.

Cette phase d'incubation s'étend, par exemple, sur 24 heures durant lesquelles la concentration en micro-organismes cibles va progressivement augmenter. Dans les dix premières heures par exemple, la concentration en micro-organismes cibles est trop faible pour interagir avec le support de capture et/ou concentration. Ainsi, durant ces dix premières heures, et tel qu'indiqué précédemment, il est préférable de maintenir le dispositif de capture et/ou concentration à l'écart de l'ensemble composé de l'échantillon et du milieu de culture afin de préserver son intégrité et éviter une détérioration des capacités dudit support en raison des composés non spécifiques contenus dans l'échantillon. Au bout de dix heures, le support de capture et concentration (104) est mis en contact de l'échantillon (4) par l'action du moyen de pression sur le guide fluidique et des moyens de déplacement qui poussent l'échantillon vers le dispositif de capture. L'échantillon passe dans le support de capture selon un flux tangentiel avant de ressortir via l'orifice de sortie (33) dans le récipient (3). Le moyen de pression et les moyens de déplacement se sont éloignés du conteneur afin de permettre au guide fluidique de reprendre sa forme initiale. Le cycle peut recommencer, le moyen de pression peut à nouveau pincer le guide fluidique et les moyens de déplacement peuvent déplacer le volume d'échantillon emprisonné dans le guide fluidique vers le dispositif de capture. La circulation répétée de l'échantillon à travers le support de capture permet d'augmenter l'efficacité de la capture des microorganismes cibles.

Dans le cas où la détection est effectuée directement sur le support de capture/concentration, le technicien peut lire le résultat de l'analyse sur le support capture/concentration à travers le dispositif de capture/concentration (99).

### Description détaillée d'un mode particulier du dispositif de capture/concentration

La cartouche (100) telle que représentée à la **figure 6** comporte un couvercle (101) et un fond (102) assemblables par des clips (103) permettant d'assurer l'étanchéité. Le support de capture (104) est disposé à l'intérieur du module réactionnel (105) de la cartouche. Le support de capture est une membrane.

La cartouche comprend :
- une voie d'admission (201) de l'échantillon liquide et/ou du liquide de lavage et/ou de liquide de lyse permettant son (leur) entrée à l'intérieur du module,
- une voie d'évacuation (202) de l'échantillon liquide et/ou du liquide de lavage et/ou de liquide de lyse et/ou du liquide d'élution.

Le support de capture et/ou concentration (104) est disposé entre la voie d'admission (201) et la voie d'évacuation (202) de telle sorte que tout ou partie de l'échantillon ou des liquides le traverse.

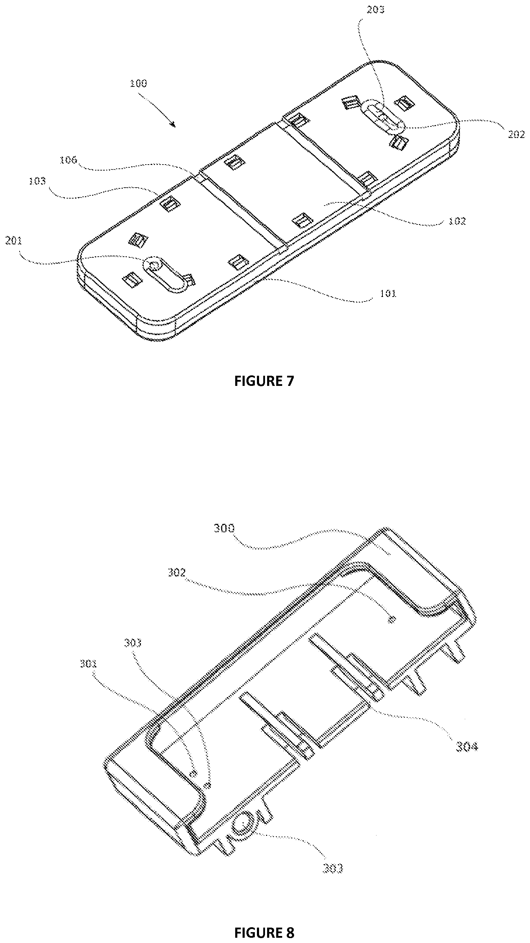

**La** **figure 7** est une vue de dessous de la cartouche représentée figure 6. La cartouche (100) est en position fermée, le fond (102) et le couvercle (101) sont assemblés par l'intermédiaire de clips (103).

La face externe du fond (102) comprend des glissières (106) servant de moyen d'insertion permettant à la cartouche d'être insérée dans un porte-cartouche.

Les glissières peuvent jouer aussi le rôle de détrompeur.

La cartouche comprend :
- une voie d'admission (201) de l'échantillon liquide et/ou du liquide de lavage et/ou de liquide de lyse permettant son (leur) entrée à l'intérieur du module,
- une voie d'évacuation (202) de l'échantillon liquide et/ou du liquide de lavage et/ou de liquide de lyse et/ou du liquide d'élution. Un bossage oblong (203) permet d'élargir la voie d'évacuation.

Le support de capture et/ou concentration (104) (non visible sur la figure 2) est disposé à l'intérieur de la cartouche entre la voie d'admission (201) et la voie d'évacuation (202) de telle sorte que tout ou partie de l'échantillon ou des liquides le traverse.

Le porte-cartouche (300) tel que représenté à la **figure 8** comprend
- un premier conduit d'admission (301) permettant l'entrée d'un échantillon liquide
- un deuxième conduit d'admission (303) permettant l'entrée d'un liquide de lavage et/ou d'un liquide de lyse et/ou un liquide d'élution
- un conduit d'évacuation (302) permettant la sortie de l'échantillon liquide et/ou du liquide de lavage ou de lyse
- un moyen d'insertion (304) permettant à la cartouche d'être insérée dans le porte-cartouche.

Le moyen d'insertion du porte cartouche peut jouer aussi le rôle de détrompeur et évite le retrait accidentel. Il est représenté ici par des glissières (304).

Le porte-cartouche (300) est destiné à être accolé à la face externe de la paroi du récipient.

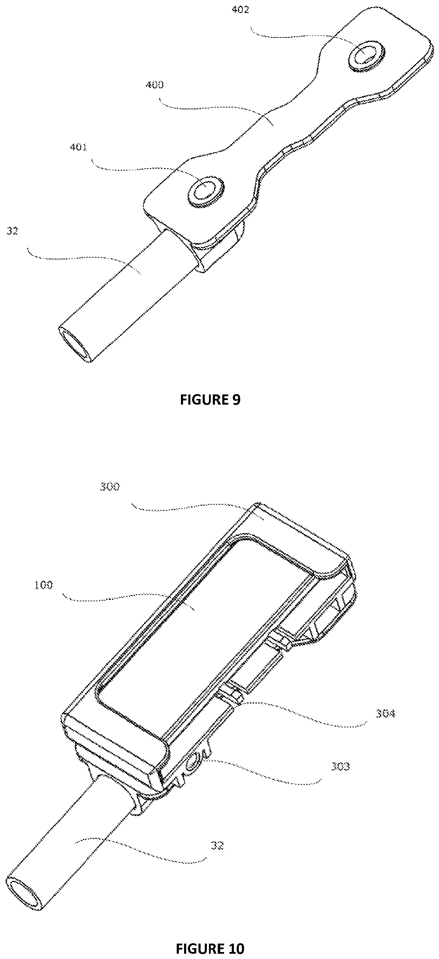

La base (400) telle que représentée **figure 9** est destinée à être accolée à la face interne de la paroi du récipient.

Elle comprend :
- au moins un canal d'admission (401) de l'échantillon liquide en liaison fluidique avec la voie d'admission de l'échantillon de la cartouche (201 figure 7) via le premier conduit d'admission du porte-cartouche (301 figure 8)
- au moins un canal d'évacuation (402) de l'échantillon liquide et/ou de lavage et/ou de lyse, en liaison fluidique avec la voie d'évacuation de la cartouche (202 figure 7) via le conduit d'évacuation du porte-cartouche (302 figure 8).

La base (400) est connectée à un guide fluidique (32) permettant à l'échantillon contenu dans le récipient d'arriver au canal d'admission de la base (401).

**La** **figure 10** montre la cartouche (100) disposée dans le porte-cartouche (300) selon un chemin fluidique dit de « capture/concentration ». La voie d'admission (201) (représentée figure 7) de la cartouche est en liaison fluidique avec le premier conduit d'admission (301) (représentée figure 8) du porte-cartouche. La voie d'évacuation (202) (représentée figure 7) de la cartouche est en liaison avec le conduit d'évacuation du porte-cartouche (302) (représentée figure 8) permettant ainsi à l'échantillon liquide de rentrer dans le module réactionnel (105) (figure 6) et d'en ressortir.

Un mouvement de translation de la cartouche dans le porte-cartouche en suivant les glissières (304) permet de passer d'un chemin fluidique dit de « capture/concentration » à un chemin fluidique dit de « lyse et/ou lavage et/ou élution».

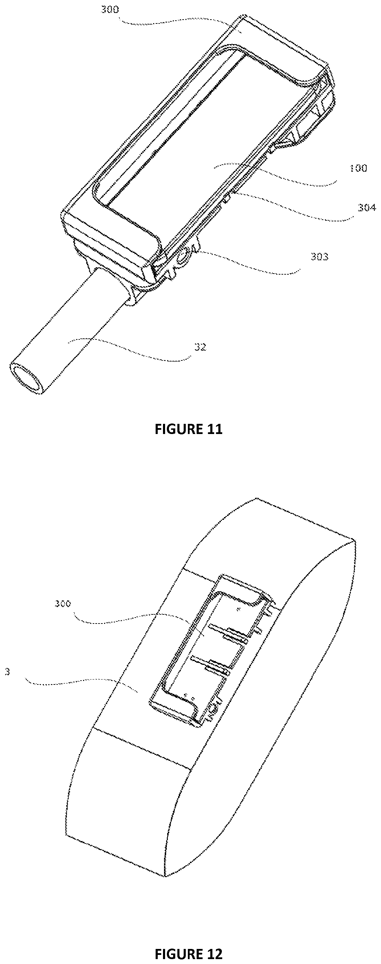

**La** **figure 11** montre la cartouche (100) disposée dans le porte-cartouche (300) selon un chemin fluidique dit de « lyse et/ou lavage et/ou élution». la voie d'admission (201) (représentée figure 7) de la cartouche est en liaison fluidique avec le deuxième conduit d'admission (303) (représentée figure 8) du porte-cartouche. La voie d'évacuation (302) (représentée figure 7) de la cartouche est en liaison avec le conduit d'évacuation (202) (représentée figure 3) du porte-cartouche permettant ainsi au liquide de lyse et/ou lavage et/ou élution de rentrer dans le module réactionnel et d'en ressortir.

Quel que soit le chemin fluidique choisi, la voie d'évacuation (202) (représentée figure 7) de la cartouche est en liaison avec le conduit d'évacuation (302) (représentée figure 8) du porte-cartouche grâce à la présence d'un bossage oblong (203) (représentée figure 7) sur la cartouche.

Selon un mode de réalisation, le porte-cartouche (300) est fixé à la face externe de la paroi du récipient (3) tel que représenté à la **figure 12****.** La cartouche peut s'insérer dans le porte-cartouche.

Le récipient peut contenir un guide fluidique qui permet de guider l'échantillon du récipient vers le canal d'admission de la base puis le premier conduit d'admission du porte-cartouche puis la voie d'admission de la cartouche.

L'échantillon traverse le support de capture/concentration puis passe à travers la voie d'évacuation de la cartouche, le conduit d'évacuation du porte-cartouche et le canal d'évacuation de la base afin de rentrer à nouveau dans le récipient.

Ainsi le dispositif de capture et/ou concentration est mis en contact avec les micro-organismes issus de l'échantillon biologique à analyser et, si les bactéries cibles sont présentes parmi lesdits micro-organismes, ces dernières se lient à leur partenaire spécifique de liaison présent au niveau dudit dispositif de capture et/ou concentration, par exemple à un anticorps fonctionnalisé.

L'échantillon passe dans le support de capture selon un flux tangentiel avant de ressortir via la voie d'évacuation dans le récipient.

La circulation répétée de l'échantillon à travers le support de pature et/ou concentration permet d'augmenter l'efficacité de la capture des microorganismes cibles.

La fonctionnalité de l'invention est illustrée avec l'exemple (non-limitatif) présenté ci-après.

### Exemple 1 :

### Efficacité de capture/concentration du dispositif selon la présente invention.

### Matériels :

- Souche Salmonella Derby réf 0904089
- Matrice : Steak haché de boeuf à 15% MG (marque Tendre et Plus)
- Matériel :
   biocapteur : membranes 550 µm (Reemay 2040) fonctionnalisées
   qPCR : kit GeneUP Salmonella (bioMérieux Cat. No.414150)
   Tampon TRIS 10 mM + 0,05 % Tween 20
   Réactif XT1 et XT2 du kit d'extraction d'Adiafood (Cat. No. ADIF9988) Bain sec pour tube eppendorf de 1,5 mL réglé à 120°C
   Tween 80
   Eau Peptonée Tamponnée (EPT) 3L (bioMérieux Cat. 42629)

### Protocole de fonctionnalisation du support de capture (membrane) :

Le support de capture est en polyester, commercialisé par la société PGI (Cat. No. Reemay 2040). Le produit est redécoupé en lamelle de 4 cm²(1 cm X 4 cm).
- le support est immergé à 37°C pendant une nuit dans une solution de BSA (Bovin Sérum Albumin)-Biotinylée à 5µg/mL;
- le support est ensuite immergé à 37°C pendant deux heures dans une solution de streptavidine à 10µg/mL
- le support est alors immergé pendant deux heures à 37°C dans une solution de partenaires de liaisons spécifiques (1 µg/mL à 40 µg/mL ; le partenaire de liaison spécifique étant une protéine de phage recombinante anti-Salmonella).

Le support sensibilisé ainsi élaboré peut être utilisé pour la détection des micro-organismes ou conservé à 2-8°C en vue d'une utilisation ultérieure.

### Préparation de l'essai :

Préparation de l'échantillon alimentaire : 375g d'échantillon alimentaire (steak haché 15% MG) sont pesés dans la poche plastique contenant la présente invention, puis 1000 mL de milieu d'enrichissement (EPT) et 5 mL de tween 80 sont ajoutés. La poche plastique est directement mise en incubation, à 39°C, dans le dispositif décrit dans la demande de brevet WO2014072438 déposée par la demanderesse mettant en oeuvre une homogénéisation douce de l'échantillon durant 5 heures d'incubation.

Programmation du cycle d'homogénéisation du dispositif :
Mode transfert latéral
Vitesse : 20 mm/s
Déplacement : 35 à 12 mm pour les 2 pales

Dans un dispositif selon l'invention tel que décrit figure 1, on dispose une membrane fonctionnalisée avec la protéine de phage anti Salmonella (membrane SLM) en tant que support de capture (échantillon Bagflow appelé B+).

On immerge également une membrane SLM. Elle sera donc en « trempage ».

Dans un autre dispositif identique, on dispose 1 membrane fonctionnalisée avec la protéine de phage anti E. coli 0157 (membrane ECO appelée B-) en tant que support de capture et jouant le rôle de témoin négatif. On immerge également une membrane ECO en témoin accrochage non spécifique. Elle sera donc en « trempage ».

### Préparation de la souche bactérienne :

A partir de la souche Salmonella Derby, réaliser une suspension à 0,6 McF puis faire des dilutions successives en tube trypsone sel de 9mL (Cat. No. AEB111499) jusqu'à la dilution 10³ CFU/mL. Réaliser un contrôle d'inoculum avec 100 µL de la dilution 10³ UFC/mL étalé sur gélose TSA (Cat. No. 43011).

Inoculer les poches plastique avec la souche de Salmonella Derby à 20 UFC/mL, soit 200 µL de la dilution 10⁵ UFC/mL.

Les poches sont homogénéisées 5 min (le temps que la souche soit répartie de façon homogène dans le sac) avant de démarrer le cycle de capture.

Pour le dispositif de la présente invention, la séquence de circulation dure 30 min à 2 mL/min, soit 60 mL d'échantillon traversent le dispositif de capture.

Pour le support en mode trempage, le temps de contact est de 30 min sous brassage.

### Traitement des supports de capture après 30 min de capture :

- Lavage des membranes avec du tampon Tris 10 mM + 0,05 % Tween 20 par trempage dans un pilulier pour les membranes dites « trempages » et pour les dispositifs, avec 3 mL de tampon connecté via une seringue au dispositif.
- Après le lavage, mettre les membranes dans des tubes eppendorfs et centrifuger 5 secondes pour évacuer le volume résiduel de tampon de lavage.
- Ajouter 100 µL du kit d'extraction sur chacune des membranes
- Incuber 20 minutes à 42°C
- Centrifuger les membranes 5 secondes pour récupérer le surnageant contenant l'ADN bactérien
- Incubée à 100°C pendant 5 minutes conformément à la notice d'utilisation du kit d'extraction.

En parallèle, l'échantillon alimentaire pré-enrichi pendant 5 heures est réparti en 5 aliquotes de 9 mL. Les aliquotes sont artificiellement contaminées avec la souche de Salmonella à une concentration allant de10⁴ à 10⁷ CFU/mL. Cette gamme permet de comparer le gain de sensibilité entre la présente invention et la méthode existante.

### Protocole GeneUP BioFire :

- Reprendre le réactif PCR avec 45 µL de tampon de reconstitution.
- Vortexer longuement puis centrifuger pour récupérer tout le liquide.
- Déposer 5 µL de réactif dans chacun des puits
- Ajouter 5 µL d'échantillon lysé
- Mettre des bouchons
- Agiter rapidement et centrifuger la plaque.
- Lancer la qPCR

### Résultats :

Contrôle d'inoculum de la suspension à 100 CFU/mL théorique : 90 CFU/mL

### Résultats qPCR :

| Echantillon | | GENE UP | |
|---|---|---|---|
| | Concentration en Salmonella (CFU/mL) | qPCR GeneUp(Ct) | Interprétation |
| B + | 20 | 29,7 | + |
| B + | 20 | 30,6 | + |
| B-(membrane ECO) | 20 | - | - |
| T + | 20 | 33,3 | + |
| T - (membrane ECO) | 20 | - | - |
| PE 10⁴ | 10 000 | - | - |
| PE 10⁵ | 100 000 | 34,3 | + |
| PE 10⁶ | 1 000 000 | 33,0 | + |
| PE 10⁷ | 10 000 000 | 31,1 | + |

Les échantillons subissant le procédé selon l'invention (B+) présentent un rendement de capture/concentration supérieur d'un log par rapport à l'échantillon Trempage. En effet, une différence de 3 Ct est observée entre les résultats (B+) et Trempage (T+). Il est ainsi démontré que le fait de faire circuler l'échantillon au travers le maillage de la membrane améliore les probabilités de capture, entre les protéines de phages et les bactéries d'intérêt.

La méthode de référence qPCR GeneUp donne un résultat positif à partir de 10⁵ CFU/mL. La méthode de capture/concentration permet donc de gagner au minimum 3 log de sensibilité sur la méthode de détection.

### Conclusion :

Cet exemple montre le gain significatif de la capture/concentration avec le dispositif selon la présente invention, par rapport à un simple trempage de la membrane dans l'échantillon. La limite de détection de la méthode (capture+ lyse + qPCR) est clairement proche de 20 UFC/mL, ce qui permet de réduire considérablement la durée d'enrichissement et donc le temps d'analyse. En effet, ce niveau de détection permet de donner une réponse positive à un échantillon de 375 g de matrice alimentaire contaminée à 1 UFC, en moins de 5h d'incubation.

## Revendications

1. Procédé de préparation d'un échantillon susceptible de contenir au moins un microorganisme cible comprenant essentiellement les étapes suivantes :
a) disposer ledit échantillon dans un conteneur (1) comprenant
- un dispositif de capture et/ou concentration (99) de microorganisme cible ou de protéine issue dudit microorganisme, comprenant un support de capture et/ou concentration (104), ledit support de capture étant une membrane poreuse ;
- un récipient (3) avec au moins deux parois dont au moins une est flexible (30), adapté pour recevoir ledit échantillon susceptible de contenir au moins un microorganisme cible, ledit récipient comprenant
o un orifice d'entrée (31) emménagé au travers d'une desdites parois et permettant le passage de l'échantillon liquide contenu dans le récipient vers le dispositif de capture et/ou concentration
o un guide fluidique d'entrée (32), susceptible de contenir un volume d'échantillon défini et permettant de guider ledit volume d'échantillon vers ledit orifice d'entrée
o au moins un moyen de contrôle du volume d'échantillon transféré du guide fluidique vers le dispositif de capture et/ou concentration au travers dudit orifice d'entrée
o un orifice de sortie (33) emménagé au travers d'une desdites parois permettant le passage de l'échantillon du dispositif de capture et/ou concentration vers le récipient,
ledit dispositif de capture/concentration était fixé à une paroi du récipient.
b) générer un déplacement de tout ou partie de l'échantillon (4) vers le guide fluidique (32),
c) générer un déplacement d'un volume défini de l'échantillon du guide fluidique vers le dispositif de capture et/ou concentration (99) au travers l'orifice d'entrée (33),
d) capturer et/ou concentrer sur le support de capture et/ou concentration ledit microorganisme cible ou une protéine issue dudit microorganisme cible contenu(e) dans l'échantillon, ledit volume d'échantillon traversant le support de capture et/ou concentration (104) puis ressortant à travers l'orifice de sortie (33) du récipient (3).
e) répéter la circulation de l'échantillon en flux tangentiel à travers le support de capture.

2. Procédé de préparation selon la revendication 1 **caractérisé en ce que** le dispositif de capture et/ou concentration (99) comprend
- un porte-cartouche (300) comprenant :
- au moins un moyen d'insertion (304) permettant à une cartouche (100) d'être insérée dans le porte-cartouche,
- au moins un premier conduit d'admission (301) permettant l'entrée d'un échantillon liquide ; le premier conduit d'admission étant en liaison avec la voie d'admission (201) de la cartouche lorsque le porte-cartouche et la cartouche sont positionnés selon un chemin fluidique dit de « capture/concentration »,
- au moins un deuxième conduit d'admission (303) permettant l'entrée d'un liquide de lavage et/ou d'un liquide de lyse et/ou un liquide d'élution ; le deuxième conduit d'admission étant en liaison avec une voie d'admission (201) de la cartouche lorsque le porte-cartouche et la cartouche sont positionnés selon un chemin fluidique dit « de lyse et/ou lavage et/ou élution».
- au moins un conduit d'évacuation (302) permettant la sortie de l'échantillon liquide et/ou du liquide de lavage ou de lyse vers le conteneur; le conduit d'évacuation étant en liaison avec la voie d'évacuation (202) de la cartouche.
- une cartouche (100) comprenant :
- au moins un moyen d'insertion (106) permettant à la cartouche d'être insérée dans au moins deux positions différentes, une première position selon un chemin fluidique dit de « capture/concentration » et une deuxième position selon un chemin fluidique dit de « lyse et/ou lavage et/ou élution».
- un module réactionnel comprenant
- au moins une voie d'admission (201) de l'échantillon liquide et/ou du liquide de lavage et/ou de lyse et/ou d'élution permettant son (leur) entrée à l'intérieur du module,
- au moins une voie d'évacuation (202) de l'échantillon liquide et/ou du liquide de lavage et/ou de lyse et/ou d'élution permettant son (leur) évacuation vers le porte-cartouche puis vers le conteneur,
- un support de capture et/ou concentration (104) disposé entre la voie d'admission et la voie d'évacuation de telle sorte que tout ou partie de l'échantillon ou des liquides le traverse.

3. Procédé selon l'une quelconque des revendications 1 ou 2 **caractérisé en ce que** le déplacement de l'échantillon vers le dispositif de capture et/ou concentration est effectué mécaniquement et de façon répétée.

4. Procédé selon l'une quelconque des revendications 1 à 3 **caractérisé en ce que** l'échantillon comprend un milieu de culture.

5. Procédé de préparation d'un échantillon selon l'une quelconque des revendications 2 à 4 comprenant également une ou plusieurs étape(s) de lavage par un liquide de lavage dispensé dans le deuxième conduit d'admission du porte-cartouche, la cartouche étant disposée selon un chemin fluidique dit de lyse et/ou lavage et/ou élution.

6. Procédé de préparation d'un échantillon selon l'une quelconque des revendications 2 à 5 comprenant également une ou plusieurs étape(s) de lyse par un liquide de lyse dispensé dans le deuxième conduit d'admission du porte-cartouche, la cartouche étant disposée selon un chemin fluidique dit de lyse et/ou lavage et/ou élution.

7. Procédé de préparation d'un échantillon selon l'une quelconque des revendications 2 à 6 comprenant une ou plusieurs étape(s) d'élution par un liquide d'élution dispensé dans le deuxième conduit d'admission du porte-cartouche, la cartouche étant disposée selon un chemin fluidique dit de lyse et/ou lavage et/ou élution.

8. Procédé de préparation d'un échantillon selon l'une quelconque des revendications 2 à 7 comprenant une étape ultérieure d'aspirer le liquide d'intérêt contenant les acides nucléiques desdits microorganismes libérés lors de la lyse.

9. Procédé selon l'une quelconque des revendications 1 à 8 **caractérisé en ce qu'**au moins 10 ml de l'échantillon contenu dans le récipient circule à travers le support de capture et/ou concentration, préférentiellement au moins 50 mL.

10. Procédé selon l'une quelconque des revendications 1 à 9 **caractérisé en ce que** tout ou partie de l'échantillon contenu dans le récipient circule à travers le support de capture et/ou concentration à un débit compris entre 0.5 mL/min par cm² de support à 5 mL/min par cm² de support, ledit support étant poreux et ayant une surface développée d'au moins 20 cm².

11. Procédé d'analyse d'un échantillon susceptible de contenir un microorganisme cible, ledit procédé mettant en oeuvre le procédé selon l'une quelconque des revendications 1 à 10, ledit procédé comprenant une étape ultérieure d'analyse dudit support de capture et/ou concentration par lecture visuelle.

12. Conteneur **caractérisé en ce qu'**il comprend
- un récipient (3) avec au moins deux parois dont au moins une est flexible (30), adapté pour recevoir ledit échantillon susceptible de contenir au moins un microorganisme cible, ledit récipient comprenant
o un orifice d'entrée (31) emménagé au travers d'une desdites parois et permettant le passage de l'échantillon liquide contenu dans le récipient vers le dispositif de capture et/ou concentration,
o un guide fluidique d'entrée (32), susceptible de contenir un volume d'échantillon défini et permettant de guider ledit volume d'échantillon vers ledit orifice d'entrée,
o au moins un moyen de contrôle du volume d'échantillon transféré du guide fluidique vers le dispositif de capture et/ou concentration au travers dudit orifice d'entrée,
o un orifice de sortie (33) emménagé au travers d'une desdites parois permettant le passage de l'échantillon du dispositif de capture et/ou concentration vers le récipient,
- un dispositif de capture et/ou concentration (99) fixé à une paroi du récipient et est en connexion avec l'intérieur dudit récipient, comprenant
o un porte-cartouche (300) comprenant :
▪ au moins un moyen d'insertion (304) permettant à une cartouche (100) d'être insérée dans le porte-cartouche,
▪ au moins un premier conduit d'admission (301) permettant l'entrée d'un échantillon liquide contenue dans le conteneur; le premier conduit d'admission étant en liaison avec la voie d'admission (201) de la cartouche lorsque le porte-cartouche et la cartouche sont positionnés selon un chemin fluidique dit de « capture/concentration »,
▪ au moins un deuxième conduit d'admission (303) permettant l'entrée d'un liquide de lavage et/ou d'un liquide de lyse et/ou un liquide d'élution ; le deuxième conduit d'admission étant en liaison avec une voie d'admission (201) de la cartouche lorsque le porte-cartouche et la cartouche sont positionnés selon un chemin fluidique dit « de lyse et/ou lavage et/ou élution»,
▪ au moins un conduit d'évacuation (302) permettant la sortie de l'échantillon liquide et/ou du liquide de lavage ou de lyse vers le conteneur; le conduit d'évacuation étant en liaison avec la voie d'évacuation (202) de la cartouche.
o une cartouche (100) comprenant :
▪ au moins un moyen d'insertion (106) permettant à la cartouche d'être insérée dans au moins deux positions différentes, une première position selon un chemin fluidique dit de « capture/concentration » et une deuxième position selon un chemin fluidique dit de « lyse et/ou lavage et/ou élution».
▪ un module réactionnel comprenant
- au moins une voie d'admission (201) de l'échantillon liquide et/ou du liquide de lavage et/ou de lyse et/ou d'élution permettant son (leur) entrée à l'intérieur du module,
- au moins une voie d'évacuation (202) de l'échantillon liquide et/ou du liquide de lavage et/ou de lyse et/ou d'élution permettant son (leur) évacuation vers le porte-cartouche puis vers le conteneur,
- un support de capture et/ou concentration (104) disposé entre la voie d'admission et la voie d'évacuation de telle sorte que tout ou partie de l'échantillon ou des liquides le traverse ; ledit support de capture et/ou concentration étant une membrane poreuse.

13. Conteneur selon 1a revendication 12 **caractérisé en ce qu'**une base (400) est accolée à la face interne de la paroi du récipient et comprenant
- au moins un canal d'admission (401) de l'échantillon liquide en liaison fluidique avec la voie d'admission de l'échantillon de la cartouche via le premier conduit d'admission du porte-cartouche,
- au moins un canal d'évacuation (402) de l'échantillon liquide et/ou de lavage et/ou de lyse, en liaison fluidique avec la voie d'évacuation de la cartouche via le conduit d'évacuation du porte-cartouche.

14. Conteneur selon la revendication 13 **caractérisé en ce que** la base est connectée à un guide fluidique permettant à l'échantillon contenu dans le récipient d'arriver au canal d'admission de la base.

15. Conteneur selon l'une quelconque des revendications 13 à 14 **caractérisé en ce que** le dispositif de capture et/ou concentration comprend :
- un moyen de récupération des acides nucléiques une fois que les microorganismes aient été lysés par le liquide de lyse, apte à être placé en communication fluidique avec le deuxième conduit d'admission du porte-cartouche.

## Patentansprüche

1. Verfahren zur Herstellung einer Probe, die potenziell mindestens einen Ziel-Mikroorganismus enthält, das im Wesentlichen die folgenden Schritte umfasst:
a) Einbringen der Probe in einen Behälter (1), umfassend:
- eine Einfang- und/oder Aufkonzentrierungsvorrichtung (99) für den Ziel-Mikroorganismus oder ein aus dem Mikroorganismus stammendes Protein, umfassend einen Einfang- und/oder Aufkonzentrierungsträger (104), wobei der Einfangträger eine poröse Membran ist,
- ein Gefäß (3) mit mindestens zwei Wänden, von denen mindestens eine flexibel (30) ist, dafür ausgelegt, die Probe aufzunehmen, die potenziell mindestens einen Ziel-Mikroorganismus enthält, wobei das Gefäß Folgendes umfasst:
o eine Einlassöffnung (31), die durch eine der Wände hindurch hergestellt ist und den Durchtritt der in dem Gefäß enthaltenen flüssigen Probe zur Einfang- und/oder Aufkonzentrierungsvorrichtung ermöglicht
o eine Einlassfluidführung (32), die ein definiertes Probenvolumen aufnehmen kann und das Leiten des Probenvolumens zur Einlassöffnung ermöglicht
o mindestens ein Mittel zum Steuern des Probenvolumens, das von der Fluidführung durch die Einlassöffnung zur Einfang- und/oder Aufkonzentrierungsvorrichtung überführt wird
o eine Auslassöffnung (33), die durch eine der Wände hindurch hergestellt ist und den Durchtritt der Probe von der Einfang- und/oder Aufkonzentrierungsvorrichtung zum Gefäß ermöglicht,
wobei die Einfang-/Aufkonzentrierungsvorrichtung an einer Wand des Gefäßes befestigt ist,
b) Bewirken einer Verlagerung der gesamten oder eines Teils der Probe (4) zur Fluidführung (32),
c) Bewirken einer Verlagerung eines definierten Volumens der Probe von der Fluidführung zur Einfang- und/oder Aufkonzentrierungsvorrichtung (99) durch die Einlassöffnung (33),
d) Einfangen und/oder Aufkonzentrieren auf dem Einfang- und/oder Aufkonzentrierungsträger und/oder Aufkonzentration des Ziel-Mikroorganismus oder eines von dem Ziel-Mikroorganismus stammenden Proteins, der bzw. das in der Probe enthalten ist, wobei das Probenvolumen den Einfang- und/oder Aufkonzentrierungsträger (104) überquert und dann durch die Auslassöffnung (33) des Gefäßes (3) wieder austritt,
e) Wiederholen des Umlaufes der Probe im Tangentialfluss über den Einfangträger.

2. Herstellungsverfahren nach Anspruch 1, **dadurch gekennzeichnet, dass** die Einfang- und/oder Aufkonzentrierungsvorrichtung (99) Folgendes umfasst
- einen Kartuschenhalter (300), umfassend:
- mindestens ein Einsetzmittel (304), das das Einsetzen einer Kartusche (100) in den Kartuschenhalter ermöglicht,
- mindestens eine erste Zulaufleitung (301), die den Eintritt einer flüssigen Probe ermöglicht, wobei die erste Zulaufleitung mit dem Zulaufweg (201) der Kartusche in Verbindung steht, wenn der Kartuschenhalter und die Kartusche in einem so genannten "Einfang-/ Aufkonzentrierungs" Fließweg positioniert sind,
- mindestens eine zweite Zulaufleitung (303), die den Eintritt einer Waschflüssigkeit und/oder einer Lyseflüssigkeit und/oder einer Elutionsflüssigkeit ermöglicht, wobei die zweite Zulaufleitung mit einem Zulaufweg (201) der Kartusche in Verbindung steht, wenn der Kartuschenhalter und die Kartusche in einem so genannten "Lyse- und/oder Wasch- und/oder Elutions"-Fließweg positioniert sind,
- mindestens eine Auslassleitung (302), die den Austritt der flüssigen Probe und/oder der Wasch- oder Lyselösung in Richtung des Behälters ermöglicht, wobei die Auslassleitung mit dem Auslassweg (202) der Kartusche in Verbindung steht,
- eine Kartusche (100), umfassend:
- mindestens ein Einsetzmittel (106), das das Einsetzen der Kartusche in mindestens zwei verschiedenen Positionen ermöglicht, einer ersten Position in einem so genannten "Einfang-/Aufkonzentrierungs"-Fließweg und einer zweiten Position in einem so genannten "Lyse- und/oder Wasch- und/oder Elutions"-Fließweg,
- ein Reaktionsmodul, umfassend
- mindestens einen Zulaufweg (201) für die flüssige Probe und/oder Wasch- und/oder Lyse- und/oder Elutionsflüssigkeit, wodurch diese in das Innere des Moduls eintreten kann bzw. können,
- mindestens einen Auslassweg (202) für die flüssige Probe und/oder Wasch- und/oder Lyse- und/oder Elutionsflüssigkeit, wodurch diese zum Kartuschenhalter und dann zum Behälter ausgleitet werden kann bzw. können,
- einen Einfang- und/oder Aufkonzentrierungsträger (104), der zwischen dem Zulaufweg und dem Auslassweg angeordnet ist, so dass die gesamte oder ein Teil der Probe oder die gesamten oder ein Teil der Flüssigkeiten diesen überqueren.

3. Verfahren nach einem der Ansprüche 1 oder 2, **dadurch gekennzeichnet, dass** die Verlagerung der Probe zur Einfang- und/oder Aufkonzentrierungsvorrichtung mechanisch und wiederholt erfolgt.

4. Verfahren nach einem der Ansprüche 1 bis 3, **dadurch gekennzeichnet, dass** die Probe ein Kulturmedium umfasst.

5. Verfahren zur Herstellung einer Probe nach einem der Ansprüche 2 bis 4, ferner umfassend einen oder mehrere Schritt(e) des Waschens mit einer Waschflüssigkeit, die in die zweite Zulaufleitung des Kartuschenhalters abgegeben wird, wobei die Kartusche in einem so genannten "Lyse- und/oder Wasch- und/oder Elutions"-Fließweg platziert ist.

6. Verfahren zur Herstellung einer Probe nach einem der Ansprüche 2 bis 5, ferner umfassend einen oder mehrere Schritt(e) der Lyse mit einer Lyseflüssigkeit, die in die zweite Zulaufleitung des Kartuschenhalters abgegeben wird, wobei die Kartusche in einem so genannten "Lyse- und/oder Wasch- und/oder Elutions"-Fließweg platziert ist.

7. Verfahren zur Herstellung einer Probe nach einem der Ansprüche 2 bis 6, umfassend einen oder mehrere Schritt(e) der Elution mit einer Elutionsflüssigkeit, die in die zweite Zulaufleitung des Kartuschenhalters abgegeben wird, wobei die Kartusche in einem so genannten "Lyse- und/oder Wasch- und/oder Elutions"-Fließweg platziert ist.

8. Verfahren zur Herstellung einer Probe nach einem der Ansprüche 2 bis 7, umfassend einen anschließenden Schritt des Absaugens der Flüssigkeit von Interesse, die die während der Lyse freigesetzten Nukleinsäuren der Mikroorganismen enthält.

9. Verfahren nach einem der Ansprüche 1 bis 8, **dadurch gekennzeichnet, dass** mindestens 10 ml der in dem Gefäß enthaltenen Probe über den Einfang- und/oder Aufkonzentrierungsträger zirkulieren, vorzugsweise mindestens 50 ml.

10. Verfahren nach einem der Ansprüche 1 bis 9, **dadurch gekennzeichnet, dass** die gesamte oder ein Teil der in dem Gefäß enthaltenen Probe über den Einfang- und/oder Aufkonzentrierungsträger mit einer Durchflussrate zwischen 0,5 ml/min pro cm² Träger bis 5 ml/min pro cm² Träger zirkuliert, wobei der Träger porös ist und eine Gesamt-Oberfläche von mindestens 20 cm² aufweist.

11. Verfahren zur Analyse einer Probe, die potenziell einen Ziel-Mikroorganismus enthält, wobei das Verfahren das Verfahren nach einem der Ansprüche 1 bis 10 umsetzt, wobei das Verfahren einen anschließenden Schritt der Analyse des Einfang- und/oder Aufkonzentrierungsträgers durch visuelles Ablesen umfasst.

12. Behälter, **dadurch gekennzeichnet, dass** er Folgendes umfasst
- ein Gefäß (3) mit mindestens zwei Wänden, von denen mindestens eine flexibel (30) ist, dafür ausgelegt, die Probe aufzunehmen, die potenziell mindestens einen Ziel-Mikroorganismus enthält, wobei das Gefäß Folgendes umfasst:
o eine Einlassöffnung (31), die durch eine der Wände hindurch hergestellt ist und den Durchtritt der der in dem Gefäß enthaltenen flüssigen Probe zur Einfang- und/oder Aufkonzentrierungsvorrichtung ermöglicht,
o eine Einlassfluidführung (32), die ein definiertes Probenvolumen aufnehmen kann und das Leiten des Probenvolumens zur Einlassöffnung ermöglicht,
o mindestens ein Mittel zum Steuern des Probenvolumens, das von der Fluidführung durch die Einlassöffnung zur Einfang- und/oder Aufkonzentrierungsvorrichtung überführt wird,
o eine Auslassöffnung (33), die durch eine der Wände hindurch hergestellt ist und den Durchtritt der Probe von der Einfang- und/oder Aufkonzentrierungsvorrichtung zum Gefäß ermöglicht,
- eine Einfang-/Aufkonzentrierungsvorrichtung (99), die an einer Wand des Gefäßes befestigt ist und mit dem Inneren des Gefäßes in Verbindung steht, umfassend
o einen Kartuschenhalter (300), umfassend:
▪ mindestens ein Einsetzmittel (304), das das Einsetzen einer Kartusche (100) in den Kartuschenhalter ermöglicht,
▪ mindestens eine erste Zulaufleitung (301), die den Eintritt einer in dem Behälter enthaltenen flüssigen Probe ermöglicht, wobei die erste Zulaufleitung mit dem Zulaufweg (201) der Kartusche in Verbindung steht, wenn der Kartuschenhalter und die Kartusche in einem so genannten "Einfang-/ Aufkonzentrierungs" Fließweg positioniert sind,
▪ mindestens eine zweite Zulaufleitung (303), die den Eintritt einer Waschflüssigkeit und/oder einer Lyseflüssigkeit und/oder einer Elutionsflüssigkeit ermöglicht, wobei die zweite Zulaufleitung mit einem Zulaufweg (201) der Kartusche in Verbindung steht, wenn der Kartuschenhalter und die Kartusche in einem so genannten "Lyse- und/oder Wasch- und/oder Elutions"-Fließweg positioniert sind,
▪ mindestens eine Auslassleitung (302), die den Austritt der flüssigen Probe und/oder der Wasch- oder Lyselösung in Richtung zum Behälter ermöglicht, wobei die Auslassleitung mit dem Auslassweg (202) der Kartusche in Verbindung steht,
o eine Kartusche (100), umfassend:
▪ mindestens ein Einsetzmittel (106), das das Einsetzen der Kartusche in mindestens zwei verschiedenen Positionen ermöglicht, einer ersten Position in einem so genannten "Einfang-/Aufkonzentrierungs"-Fließweg und einer zweiten Position in einem so genannten "Lyse- und/oder Wasch- und/oder Elutions"-Fließweg,
▪ ein Reaktionsmodul, umfassend
- mindestens einen Zulaufweg (201) für die flüssige Probe und/oder Wasch- und/oder Lyse- und/oder Elutionsflüssigkeit, wodurch diese in das Innere des Moduls eintreten kann bzw. können,
- mindestens einen Auslassweg (202) für die flüssige Probe und/oder Wasch- und/oder Lyse- und/oder Elutionsflüssigkeit, wodurch diese zum Kartuschenhalter und dann zum Behälter ausgleitet werden kann bzw. können,
- einen Einfang- und/oder Aufkonzentrierungsträger (104), der zwischen dem Zulaufweg und dem Auslassweg angeordnet ist, so dass die gesamte oder ein Teil der Probe oder die gesamten oder ein Teil der Flüssigkeiten diesen überqueren, wobei der Einfang- und/oder Aufkonzentrierungsträger eine poröse Membran ist.

13. Behälter nach Anspruch 12, **dadurch gekennzeichnet, dass** eine Basis (400) mit der Innenfläche der Wand des Gefäßes verbunden ist und Folgendes umfasst
- mindestens einen Zulaufkanal (401) für die flüssige Probe in Flüssigkeitsverbindung mit dem Probenzulaufweg der Kartusche über die erste Zulaufleitung des Kartuschenhalters,
- mindestens einen Auslasskanal (402) für die flüssige Probe und/oder zum Waschen und/oder zur Lyse in Flüssigkeitsverbindung mit dem Auslassweg der Kartusche über die Auslassleitung des Kartuschenhalters.

14. Behälter nach Anspruch 13, **dadurch gekennzeichnet, dass** die Basis mit einer Fluidführung verbunden ist, die es ermöglicht, dass die in dem Gefäß enthaltene Probe zum Zulaufkanal der Basis gelangen kann.

15. Behälter nach einem der Ansprüche 13 bis 14, **dadurch gekennzeichnet, dass** die Einfang- und/oder Aufkonzentrierungsvorrichtung Folgendes umfasst:
- ein Mittel zur Gewinnung der Nukleinsäuren, nachdem die Mikroorganismen durch die Lyseflüssigkeit lysiert worden sind, das in Flüssigkeitskommunikation mit der zweiten Zulaufleitung des Kartuschenhalters platziert werden kann.

## Claims

1. Method for preparing a sample that may contain at least one target microorganism, comprising essentially the following steps:
a) placing said sample in a container (1) comprising
- a device for the capture and/or concentration (99) of a target microorganism or of a protein from said microorganism, comprising a capture and/or concentration support (104), said capture support being a porous membrane;
- a receptacle (3) with at least two walls, at least one of which is flexible (30), capable of receiving said sample that may contain at least one target microorganism, said receptacle comprising
o an inlet orifice (31) made through one of said walls and allowing the liquid sample contained in the receptacle to pass to the capture and/or concentration device,
o an inlet fluid guide (32), capable of containing a defined sample volume and making it possible to guide said sample volume to said inlet orifice,
o at least one means for controlling the volume of sample transferred from the fluid guide to the capture and/or concentration device through said inlet orifice,
o an outlet orifice (33) made through one of said walls allowing the sample to pass from the capture and/or concentration device to the receptacle,
said capture/concentration device being attached to a wall of the receptacle;
b) generating a displacement of all or part of the sample (4) to the fluid guide (32),
c) generating a displacement of a defined volume of the sample from the fluid guide to the capture and/or concentration device (99) through the inlet orifice (33),
d) capturing and/or concentrating, on the capture and/or concentration support, said target microorganism or a protein from said target microorganism contained in the sample, said volume of sample crossing the capture and/or concentration support (104) and then exiting again through the outlet orifice (33) of the receptacle (3),
e) repeating the circulation of the sample in tangential flow through the capture support.

2. Preparation method according to Claim 1, **characterized in that** the capture and/or concentration device (99) comprises
- a cartridge holder (300) comprising:
- at least one insertion means (304) allowing a cartridge (100) to be inserted into the cartridge holder,
- at least one first inlet duct (301) allowing the entry of a liquid sample; the first intake duct being linked to the intake pathway (201) of the cartridge when the cartridge holder and the cartridge are positioned according to a "capture/concentration" fluid path,
- at least one second intake duct (303) allowing the entry of a washing liquid and/or of a lysis liquid and/or an elution liquid; the second intake duct being linked to an intake pathway (201) of the cartridge when the cartridge holder and the cartridge are positioned according to a "lysis and/or washing and/or elution" fluid path,
- at least one discharge duct (302) allowing the exit of the liquid sample and/or of the washing or lysis liquid to the container; the discharge duct being linked to the discharge pathway (202) of the cartridge;
- a cartridge (100) comprising:
- at least one insertion means (106) allowing the cartridge to be inserted in at least two different positions, a first position according to a "capture/concentration" fluid path and a second position according to a "lysis and/or washing and/or elution" fluid path,
- a reaction module comprising
- at least one intake pathway (201) for the liquid sample and/or the washing and/or lysis and/or elution liquid allowing it (them) to enter the module,
- at least one discharge pathway (202) for the liquid sample and/or the washing and/or lysis and/or elution liquid allowing it (them) to be discharged to the cartridge holder and then to the container,
- a capture and/or concentration support (104) placed between the intake pathway and the discharge pathway such that all or part of the sample or of the liquids crosses it.

3. Method according to either one of Claims 1 and 2, **characterized in that** the displacement of the sample to the capture and/or concentration device is carried out mechanically and repeatedly.

4. Method according to any one of Claims 1 to 3, **characterized in that** the sample comprises a culture medium.

5. Method for preparing a sample according to any one of Claims 2 to 4, also comprising one or more step(s) of washing with a washing liquid dispensed in the second inlet duct of the cartridge holder, the cartridge being placed according to a "lysis and/or washing and/or elution" fluid path.

6. Method for preparing a sample according to any one of Claims 2 to 5, also comprising one or more step(s) of lysis with a lysis liquid dispensed in the second intake duct of the cartridge holder, the cartridge being placed according to a "lysis and/or washing and/or elution" fluid path.

7. Method for preparing a sample according to any one of Claims 2 to 6, comprising one or more step(s) of elution with an elution liquid dispensed in the second intake duct of the cartridge holder, the cartridge being placed according to a "lysis and/or washing and/or elution" fluid path.

8. Method for preparing a sample according to any one of Claims 2 to 7, comprising a subsequent step of suctioning the liquid of interest containing the nucleic acids of said microorganisms released during the lysis.

9. Method according to any one of Claims 1 to 8, **characterized in that** at least 10 ml of the sample contained in the receptacle circulates through the capture and/or concentration support, preferentially at least 50 ml.

10. Method according to any one of Claims 1 to 9, **characterized in that** all or part of the sample contained in the receptacle circulates through the capture and/or concentration support at a flow rate of between 0.5 ml/min per cm² of support and 5 ml/min per cm² of support, said support being porous and having a developed surface area of at least 20 cm².

11. Method for analysing a sample that may contain a target microorganism, said method implementing the method according to any one of Claims 1 to 10, said method comprising a subsequent step of analysing said capture and/or concentration support by visual reading.

12. Container **characterized in that** it comprises
- a receptacle (3) with at least two walls, at least one of which is flexible (30), capable of receiving said sample that may contain at least one target microorganism, said receptacle comprising
o an inlet orifice (31) made through one of said walls and allowing the liquid sample contained in the receptacle to pass to the capture and/or concentration device,
o an inlet fluid guide (32), capable of containing a defined sample volume and making it possible to guide said sample volume to said inlet orifice,
o at least one means for controlling the volume of sample transferred from the fluid guide to the capture and/or concentration device through said inlet orifice,
o an outlet orifice (33) made through one of said walls allowing the sample to pass from the capture and/or concentration device to the receptacle,
- a capture and/or concentration device (99) attached to a wall of the receptacle and connected to the inside of said receptacle, comprising
o a cartridge holder (300) comprising:
▪ at least one insertion means (304) allowing a cartridge (100) to be inserted into the cartridge holder,
▪ at least one first inlet duct (301) allowing the entry of a liquid sample contained in the container; the first intake duct being linked to the intake pathway (201) of the cartridge when the cartridge holder and the cartridge are positioned according to a "capture/concentration" fluid path,
▪ at least one second intake duct (303) allowing the entry of a washing liquid and/or of a lysis liquid and/or an elution liquid; the second intake duct being linked to an intake pathway (201) of the cartridge when the cartridge holder and the cartridge are positioned according to a "lysis and/or washing and/or elution" fluid path,
▪ at least one discharge duct (302) allowing the exit of the liquid sample and/or of the washing or lysis liquid to the container; the discharge duct being linked to the discharge pathway (202) of the cartridge;
o a cartridge (100) comprising:
▪ at least one insertion means (106) allowing the cartridge to be inserted in at least two different positions, a first position according to a "capture/concentration" fluid path and a second position according to a "lysis and/or washing and/or elution" fluid path,
▪ a reaction module comprising
- at least one intake pathway (201) for the liquid sample and/or the washing and/or lysis and/or elution liquid allowing it (them) to enter the module,
- at least one discharge pathway (202) for the liquid sample and/or the washing and/or lysis and/or elution liquid allowing it (them) to be discharged to the cartridge holder and then to the container,
- a capture and/or concentration support (104) placed between the intake pathway and the discharge pathway such that all or part of the sample or of the liquids crosses it; said capture and/or concentration support being a porous membrane.

13. Container according to Claim 12, **characterized in that** a base (400) is placed right next to the internal face of the wall of the receptacle and comprising
- at least one channel for intake (401) of the liquid sample, fluidly linked to the sample intake pathway of the cartridge via the first intake duct of the cartridge holder,
- at least one channel for discharge (402) of the liquid sample and/or washing and/or lysis, fluidly linked to the discharge pathway of the cartridge via the discharge duct of the cartridge holder.

14. Container according to Claim 13, **characterized in that** the base is connected to a fluid guide which allows the sample contained in the receptacle to reach the intake channel of the base.

15. Container according to either one of Claims 13 and 14, **characterized in that** the capture and/or concentration device comprises:
- a means for recovering the nucleic acids once the microorganisms have been lysed by the lysis liquid, said recovering means being able to be placed in fluid communication with the second intake duct of the cartridge holder.
